# EUROPEAN PATENT APPLICATION

(11) **EP 2 275 545 A1**
(43) Date of publication of application: **19.01.2011**
(21) Application number: 09075314.6
(22) Date of filing: 16.07.2009
(51) Int. Cl.: C12N 15/113, C07K 14/47

(54) **Use of microRNA-24 and/or its targets for the treatment and prevention of ischemia and induction of angiogenesis**

(71) Applicant: Julius-Maximilians-Universität Würzburg, 97070 Würzburg (DE)
(72) Inventor: Thum, Thomas, 97082 Würzburg (DE); Fiedler, Jan, 97204 Höchberg (DE)
(74) Representative: Engelhard, Markus

(57) **Abstract**

The present invention relates to the use of GATA2, PAK4, RASA1 and/or a modulator, in particular an inhibitor of miR-24 for the manufacture of a medicament for the treatment and/or prevention of ischemia and for the induction of angiogenesis.

The present invention also relates to an *in vitro* method for diagnosing ischemia or prevalence for ischemia, to a method for identifying a modulator of miR-24, GATA2, PAK4 and/or RASA1. In addition the present invention relates to a kit comprising an antagomir and/or an antisense oligonucleotide essentially complementary to SEQ ID NO: 1, to endothelial cells devoid of expressing functional miR-24, and to a non-human, transgenic animal comprising these endothelial cells.

SEQUENCE LISTING

<110> Julius-Maximilians-Universität Würzburg

<120> Use of microRNA-24 targets for the treatment and prevention of
ischemia and induction of angiogenesis

<130> U30044

<160> 14

<170> PatentIn version 3.3

<210> 1
<211> 22
<212> DNA
<213> Artificial

<220>
<223> Antagomir-24: 5'-CTGTTCCTGCTGAACTGAGCCA-chol-3'

<220>
<221> chol-3'
<222> (3)..(3)
<223> at the 3'-end of antagomir

<400> 1
ctgttcctgc tgaactgagc ca 22

<210> 2
<211> 23
<212> DNA
<213> Artificial

<220>
<223> scrambled Antagomir: 5'-ACAAACACCAUUGUCACACUCCA-chol-3'

<220>
<221> chol-3'
<222> (3)..(3)
<223> at the 3'-end of scrambled Antagomir

<400> 2
acaaacacca uugucacacu cca 23

<210> 3
<211> 22
<212> RNA
<213> Homo sapiens

<400> 3
uggcucaguu cagcaggaac ag 22

<210> 4
<211> 22
<212> RNA
<213> Mus musculus

<400>  4
uggcucaguu cagcaggaac ag                                                22

<210>  5
<211>  20
<212>  DNA
<213>  Artificial

<220>
<223>  Forward Primer BAMBI

<400>  5
tctcaggttt tggagggaga                                                   20

<210>  6
<211>  20
<212>  DNA
<213>  Artificial

<220>
<223>  Reverse Primer BAMBI

<400>  6
ggccgagact gacactcaat                                                   20

<210>  7
<211>  20
<212>  DNA
<213>  Artificial

<220>
<223>  Forward Primer ESM1

<400>  7
caagtgatat gccagggtca                                                   20

<210>  8
<211>  20
<212>  DNA
<213>  Artificial

<220>
<223>  Reverse Primer ESM1

<400>  8
tggttgtttt gcatgaggac                                                   20

<210>  9
<211>  20
<212>  DNA
<213>  Artificial

<220>
<223>  Forward Primer HO-1

<400>  9
catcaccaga cccagacaga                                                   20

<210> 10
<211> 20
<212> DNA
<213> Artificial

<220>
<223> Reverse Primer HO-1

<400> 10
aaggccgact ttaagggaag 20


<210> 11
<211> 23
<212> DNA
<213> Artificial

<220>
<223> Forward Primer NTN4

<400> 11
gagccagtta ttcagcaaag aaa 23


<210> 12
<211> 20
<212> DNA
<213> Artificial

<220>
<223> Reverse Primer NTN4

<400> 12
atgcagaggc catgctaatc 20


<210> 13
<211> 20
<212> DNA
<213> Artificial

<220>
<223> Forward Primer SIRT1

<400> 13
ggagtcacag tgtgccagaa 20


<210> 14
<211> 20
<212> DNA
<213> Artificial

<220>
<223> Reverse Primer SIRT1

<400> 14
ccttcctttc tagcgtgagc 20

## Description

### Field of the invention

The present invention relates to the field of microRNA, in particular to the use of microRNAs, their targets and microRNA modulators, in particular inhibitors for the diagnosis, prevention and/or therapy. Especially, the present invention relates to the use of modulators, in particular inhibitors, targets and precursors of miR-24 for the treatment and/or prevention of ischemia and angiogenesis associated with cancer and for the induction of angiogenesis. The present invention also relates to an *in vitro* method for diagnosing ischemia or prevalence for ischemia and to a method for identifying a modulator of miR-24, GATA2, PAK4 and/or RASA1. In addition, the present invention relates to a kit comprising an antagomir and/or an antisense oligonucleotide essentially complementary to SEQ ID NO: 1, endothelial cells devoid of expressing functional miR-24, and a non-human, transgenic animal comprising these endothelial cells.

### Background of the invention

MicroRNAs (miRNAs) are endogenous small non-coding single stranded RNAs that control diverse biological processes and major signaling pathways, like developmental timing, hematopoietic cell differentiation, apoptosis, cell proliferation, and organ development. They regulate the expression of complementary target mRNAs by post-transcriptional gene silencing, which leads to mRNA cleavage or translational repression. With more than 200 members per species in higher eukaryotes, miRNAs are one of the largest gene families accounting for about 1 % of the genome (Bartel, 2004). More than one third of all human genes are targeted by miRNAs. MiRNAs and their targets seem to form complex regulatory networks. For example, a single miRNA regulates many different mRNA targets, and several different miRNAs control a single mRNA target. Consequently, the unique combination of miRNAs that are expressed in each cell type might affect the utilization of thousands of mRNAs.

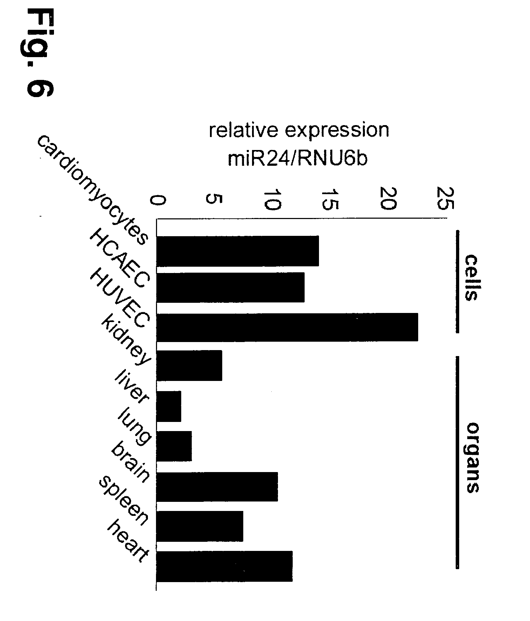

MiRNAs were recently implicated in the regulation of diverse cardiac functions in a series of genetic studies (Care et al., 2007). Myocardial infarction results in hypoxia of cardiac tissue that triggers an array of pathophysiological effects including cardiomyocyte apoptosis and impairment of vascularization. Studies have shown miRNAs to be important for regulation of endothelial function, especially angiogenesis (Wang et al., 2008). Although these studies help to delineate the role of miRNA in heart physiology, growth and morphogenesis, molecular mechanisms for miRNAs in cardiac disease pathways are poorly understood. MiR-24 is expressed in a variety of organs (Fig. 6), but its role in the cardiovascular system is unclear. Therefore, the therapeutic potential of specific miRNAs and their antagonists in cardiac diseases remains to be established.

Myocardial infarction and cardiac dysfunction represent a critical health burden worldwide (http://www.who.int/whosis/whostat/2009/en/index.html, WHO, 2009). Due to the increased life expectancy, incidence and prevalence of cardiovascular diseases will rise. Cardiac ischemia triggers left ventricular remodeling and development of heart failure, and the prognosis of heart failure is as bad as for certain malignant tumors (Hill et al., 2008). A central issue of ventricular remodeling after myocardial infarction is an insufficient angiogenesis. Therapies improving neovascularization after myocardial infarction favorably affect cardiac outcome, however, such approaches are scarce and mechanistically not well understood.

Therefore, therapies for the improvement in particular of cardiac healing processes post cardiovascular diseases have to be developed.

The solution to this problem is achieved by providing the embodiments characterized by the claims, and described further below.

### Summary of the invention

A first aspect of the present invention relates to the use of GATA2, PAK4, RASA1 and/or a modulator, in particular an inhibitor of microRNA-24 (miR-24) for the manufacture of a medicament for the treatment and/or prevention of ischemia.

A further aspect of the present invention relates to the use of GATA2, PAK4, RASA1 and/or a modulator, in particular an inhibitor of miR-24 for the manufacture of a medicament for the induction of angiogenesis.

A further aspect of the present invention relates to the use of a precursor of miR-24 (pre-miR-24) for the manufacture of a medicament for the treatment of angiogenesis associated with cancer.

A further aspect of the present invention relates to an *in vitro* method for diagnosing ischemia or prevalence for ischemia, comprising the steps of:
a) providing a test sample of a subject comprising endothelial cells;
b) identifying the amount of miR-24, GATA2, PAK4 and/or RASA1 in the test sample;
c) comparing the amount of miR-24, GATA2, PAK4 and/or RASA1 in the test sample with a control sample;
   wherein an up-regulation of miR-24 and/or a down-regulation of GATA2, PAK4 and/or RASA1 in the test sample indicates ischemia or prevalence for ischemia.

A further aspect of the present invention relates to a method for identifying a modulator of miR-24, GATA2, PAK4 and/or RASA1 comprising the steps of:
a) providing a cell culture expressing miR-24, GATA2, PAK4 and/or RASA1;
b) contacting a candidate substance with the cell culture;
c) assessing the activity and/or expression of miR-24, GATA2, PAK4 and/or RASA1;
d) comparing the expression and/or activity of miR-24, GATA2, PAK4 and/or RASA1 of step c) with the expression and/or activity in the absence of the candidate compound,
   wherein a difference in the expression and/or activity of miR-24, GATA2, PAK4 and/or RASA1 qualifies the candidate substance as a modulator of miR-24, GATA2, PAK4 and/or RASA1.

A further aspect of the present invention relates to a kit comprising an antagomir and/or an antisense oligonucleotide essentially complementary to SEQ ID NO: 1.

A further aspect of the present invention relates to an endothelial cell devoid of expressing functional miR-24.

A further aspect of the present invention relates to a non-human, transgenic animal comprising cells devoid of expressing functional miR-24.

A further aspect of the present invention relates to GATA2, PAK4, RASA1 and/or a modulator, in particular an inhibitor of microRNA-24 (miR-24) for use as a medicament for the treatment and/or prevention of ischemia.

A further aspect of the present invention relates to GATA2, PAK4, RASA1 and/or a modulator, in particular an inhibitor of microRNA-24 (miR-24) for use as a medicament for the induction of angiogenesis.

In a further aspect the present invention relates to a method for treating ischemia in a subject in need thereof, comprising the steps of:
a) identifying a subject suffering from ischemia;
b) inhibiting the expression of miR-24 and/or activating the expression of GATA2, PAK4 and/or RASA1 in endothelial cells of the subject.

In a still further aspect, the invention relates to a method for preventing ischemia in a subject which is at risk of developing ischemia, comprising the steps of:
a) identifying a subject which is at risk of developing ischemia;
b) inhibiting the expression of miR-24 and/or activating the expression of GATA2, PAK4 and/or RASA1 in endothelial cells of the subject.

The invention will be more apparent from the disclosure of the following description together with the figures and sequence listing.

### Brief description of the drawings

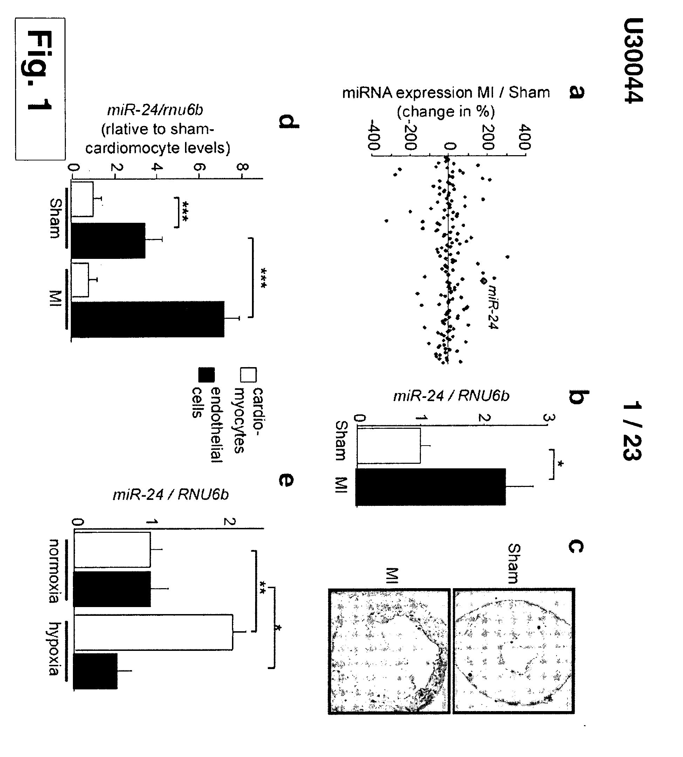

**Fig. 1** **displays a selective miR-24 upregulation in endothelial cells after myocardial infarction. (a)** Microarray-based analysis of miRNAs after chronic myocardial infarction (ten weeks) versus Sham-operated rats (each n=3 arrays per group). MiR-24 is shown as larger rhomb. **(b)** Quantitative real-time-PCR of miR-24 and RNU6b in left ventricular tissue (border zone) of mice fourteen days after myocardial infarction or Sham-controls (n=6-7 per group). **(c)** *In situ* hybridization of miR-24 in infarcted and non-infarcted mouse myocardium. **(d)** Expression of miR-24 relative to RNU6b in fractionated endothelial cells (magnetic affinity cell sorted CD146-positive cells) and cardiomyocytes after myocardial infarction of mice (n=4 per group). **(e)** Expression of miR-24 / RNU6b in human umbilical vein endothelial cells (HUVEC) and rat neonatal cardiomyocytes 24 h after normoxic (21 % 02) or hypoxic conditions (1 % 02).

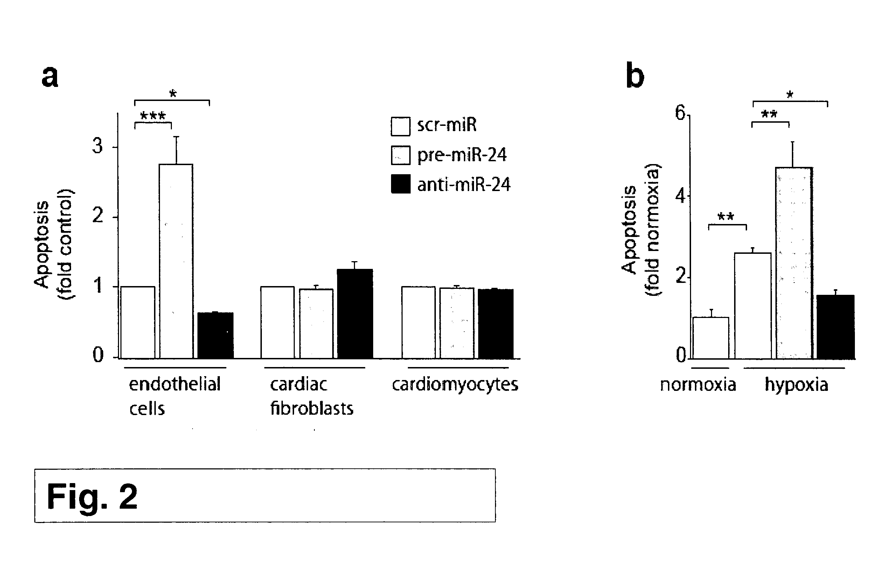

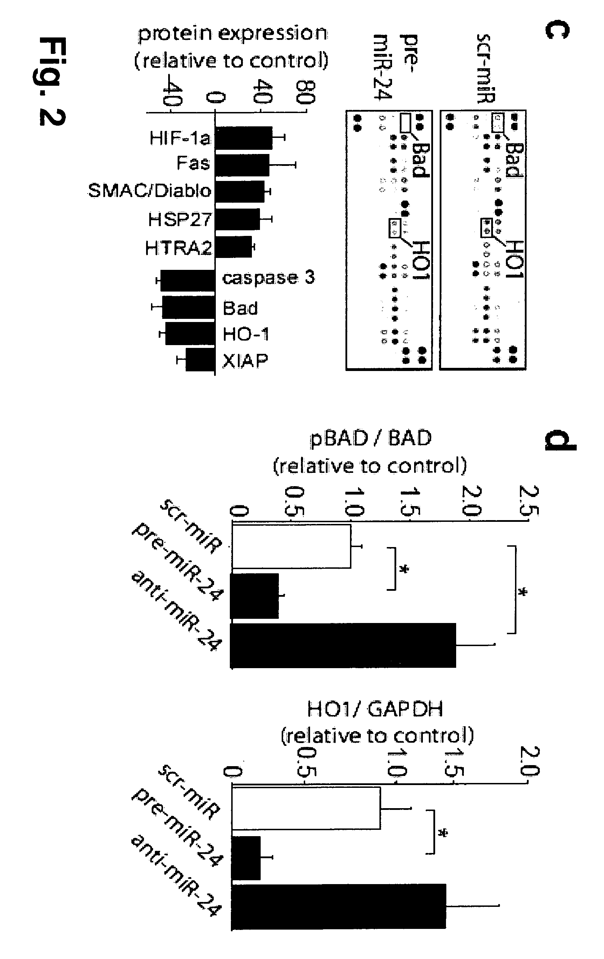

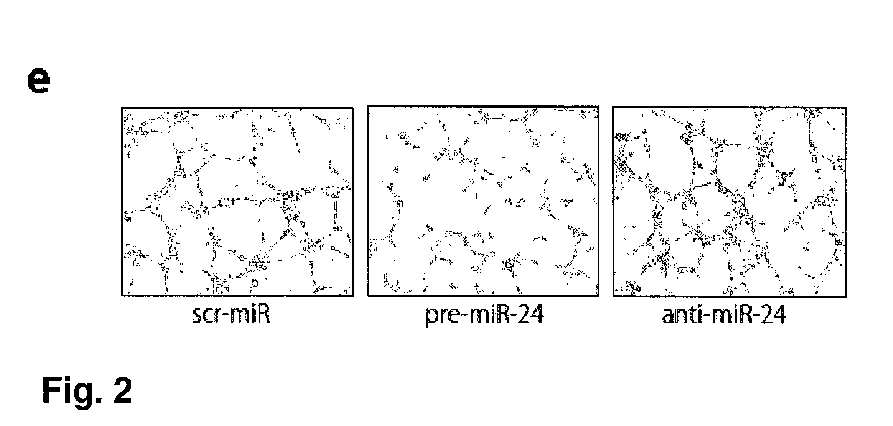

**Fig. 2** **displays the activation of apoptotic programs in endothelial cells and impairment of tube formation capacity by miR-24. (a)** Relative changes of apoptotic cells 72 h after transfection of endothelial cells (HUVECs), neonatal rat cardiomyocytes or rat cardiac fibroblasts with scrambled-miR (scr-miR), synthetic mir-24 precursors (pre-miR-24) or miR-24 antagonists (anti-miR-24). **(b)** Number of apoptotic endothelial cells after transfection with scr-miR, pre-miR-24 or anti-miR-24 for 72 h and subsequent exposure to hypoxia (1 % O₂, 24 h). **(c)** Proteome ProfilerTM Array membranes (upper) and statistical summary (lower) after hybridization of endothelial protein extracts 72 h post-transfection with scrambled-miRNAs (scr-miR) or synthetic miR-24 precursors (pre-miR-24). The signal intensity of 35 different apoptosis-related proteins (in duplicates) is shown. **(d)** Ratio of phospho-BAD relative to BAD expression (left) and heme oxygenase 1 (HO-1) expression in endothelial cells 72 h after transfection with scr-miR, pre-miR-24 or anti-miR-24. **(e)** Tube formation capacity of HUVECs 24 h after seeding on top of matrigels and 72 h post transfection with scr-miR, pre-miR-24 or anti-miR-24.

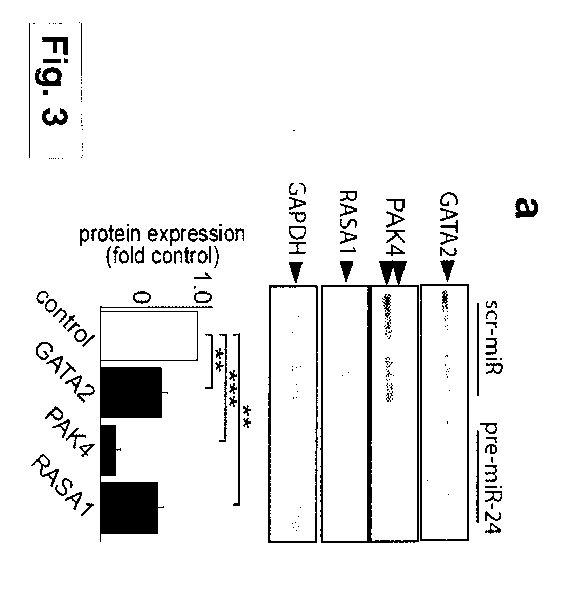

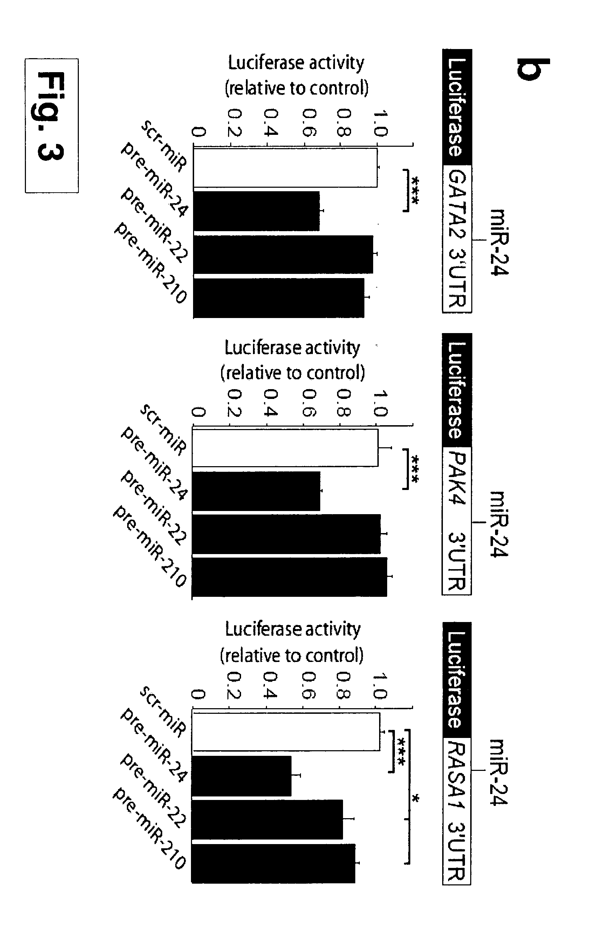

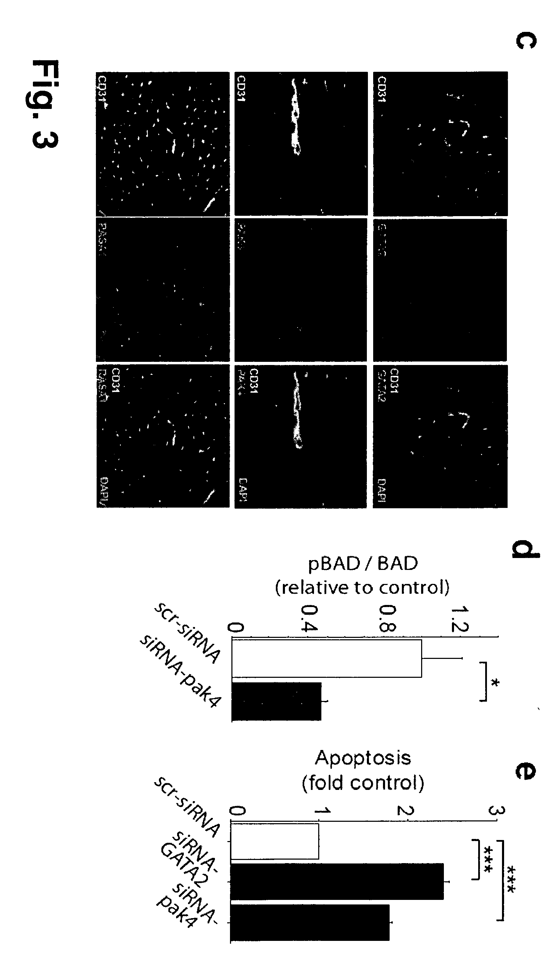

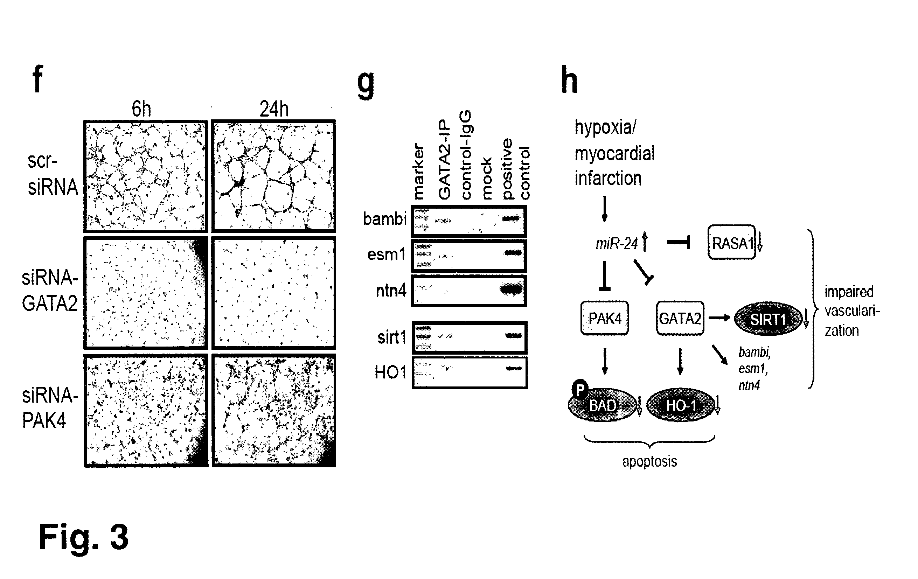

**Fig. 3** **displays that miR-24 coordinates a complex program of endothelial-enriched transcription factors and kinases that are important for endothelial apoptosis and vascularization capacity. (a)** Protein expression of GATA2, PAK4, RASA1 and GAPDH 72 h after transfection with scrambled miRNAs (scr-miR) or synthetic miR-24 precursors (pre-miR-24) and statistical summary. **(b)** Activities of luciferase reporter constructs comprising the 3'UTR region of GATA2, PAK4 or RASA1 mRNA after transfection of several synthetic miRNAs. **(c)** Localization of CD31, GATA2, PAK4 and RASA1 in sections of mouse hearts. Nuclei are counterstained with 4',6-diamidin-2'-phenylindol-dihydrochlorid (DAPI). **(d)** Ratios of pBAD (measured by pBAD-specific ELISA) and total BAD (measured by Western blotting) in endothelial cells 48 h after transfection of scrambled siRNA (scr-siRNA) or siRNA against PAK4 (siRNA-PAK4). **(e)** Apoptosis of endothelial cells after transfection of scrambled siRNA (scr-siRNA) or siRNAs specific for GATA2 (siRNA-GATA2) or PAK4 (siRNAPAK4). **(f)** Tube formation of HUVECs after transfection of scr-siRNA or siRNA against GATA2 or PKA4 6 h and 24 h after seeding on top of matrigels. **(g)** Chromatin immunoprecipitation of several DNA sequences by GATA2 when compared to appropriate controls. **(h)** Scheme of miR-24 function in endothelial cells.

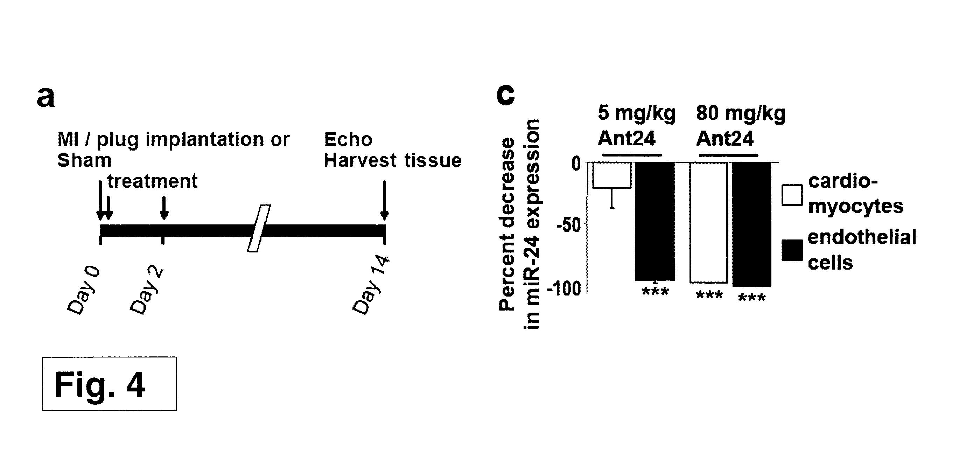

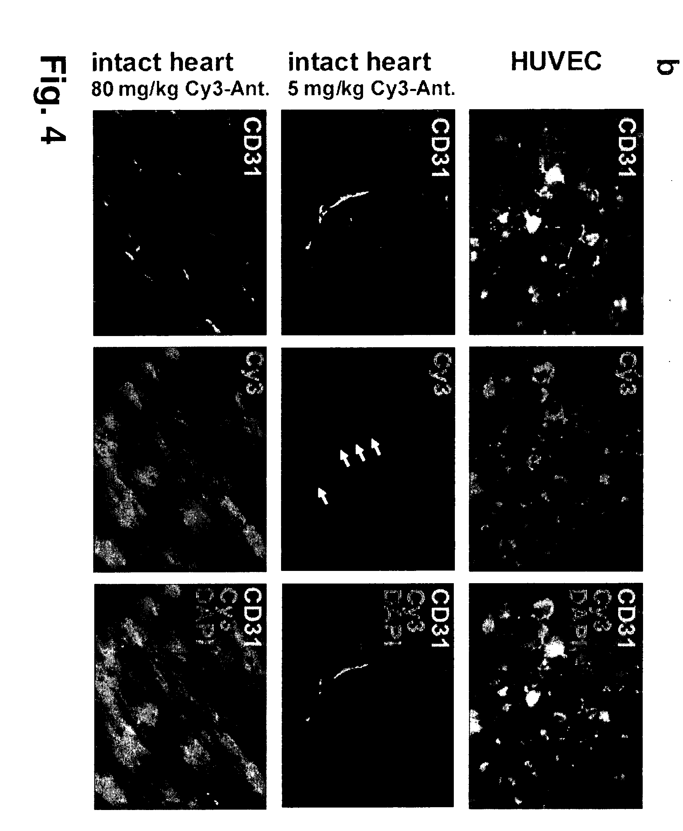

**Fig. 4** **displays specific targeting of cardiac endothelial cells by low antagomir concentrations. (a)** Design of the antagomir *in vivo* study. **(b)** Uptake of Cy3- labeled antagomirs in endothelial cells *in vitro* and *in vivo.* Cellular uptake of Cy3-labeled antagomirs (10µg/ml, 24 h) in HUVECs (top row). Primary deposition of Cy3- labeled antagomirs closely to cardiac blood vessels after injection of a low dose (5 mg/kg; middle row) or massive cardiac uptake after injection of high antagomir doses (80 mg/kg, bottom row). **(c)** Detection of miR-24 levels in fractionated cardiac endothelial cells and cardiomyocytes after treatment with two injections of 80 mg/kg (high dose) or 5 mg/kg (low dose) antagomir-24.

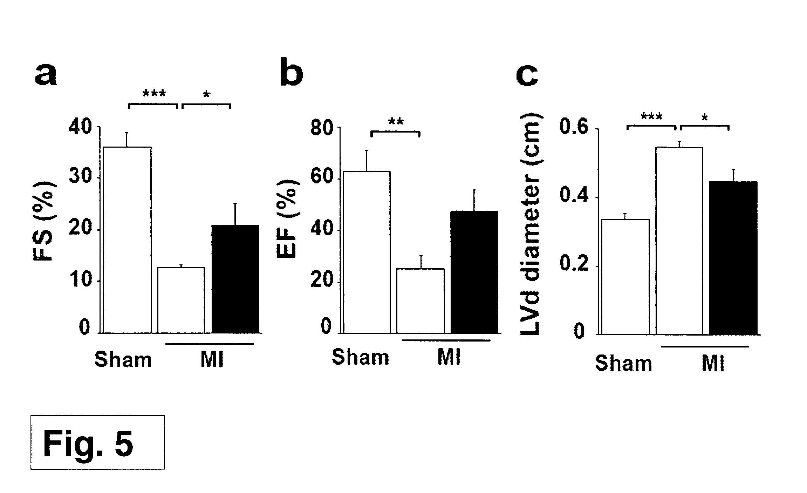

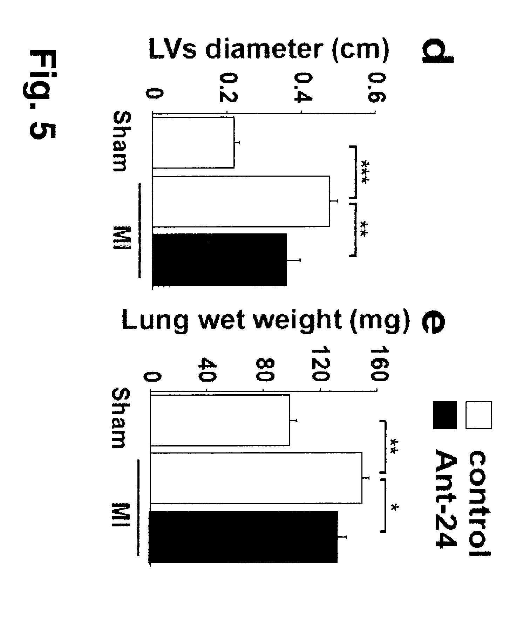

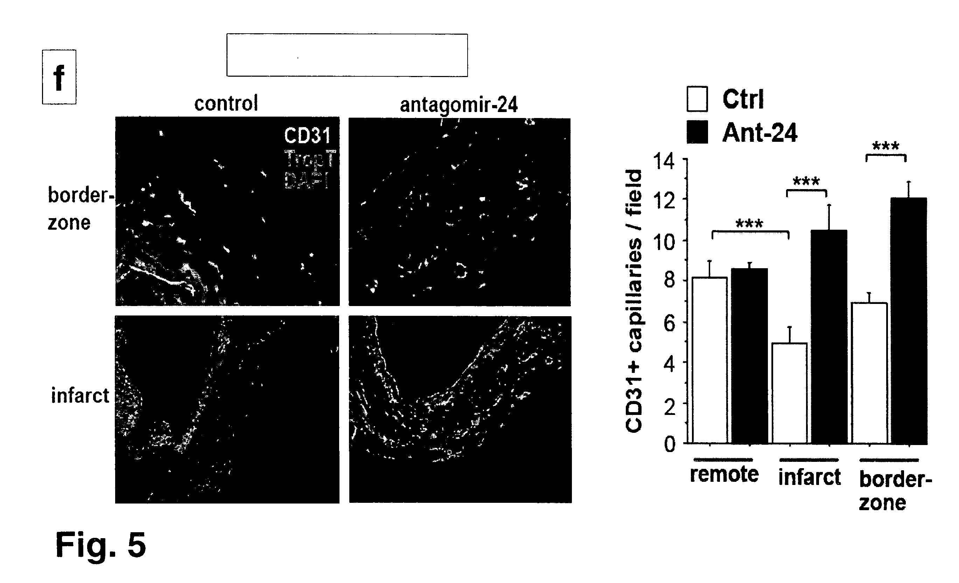

**Fig. 5** **displays that antagomir-24 treatment improves vascularization and preserves cardiac function after myocardial infarction. (a)** Cardiac function (left, fractional shortening (FS) measured by echocardiography fourteen days after Sham-operation or myocardial infarction (MI). **(b)** Ejection fraction (EF) measured by conductance catheter-based invasive hemodynamics. **(c)** Diastolic and **(d)** systolic left ventricular diameter (LVd and LVs) and **(e)** lung wet weight after Sham-operation or myocardial infarction. **(f)** Detection of CD31 in endothelium, troponin T in surviving cardiomyocytes and nuclei (DAPI) in cardiac sections of the border zone and infarct zone fourteen days after myocardial infarction and after treatment with antagomirs against a scrambled sequence (control) or miR-24 (antagomir-24). Statistical summary of the number of CD31-postive vessels in the infarct zone, border zone and remote area of the infarct (right).
**Fig. 6** **displays miR-24 expression in various cells and organs.** Quantitative realtime-PCR-determined expression of miR-24 relative to RNU6b in isolated RNA from human cardiovascular cells (cardiomyocytes, human coronary arterial endothelial cells (HCAEC) and human umbilical vein endothelial cells (HUVEC)) and diverse human organs.

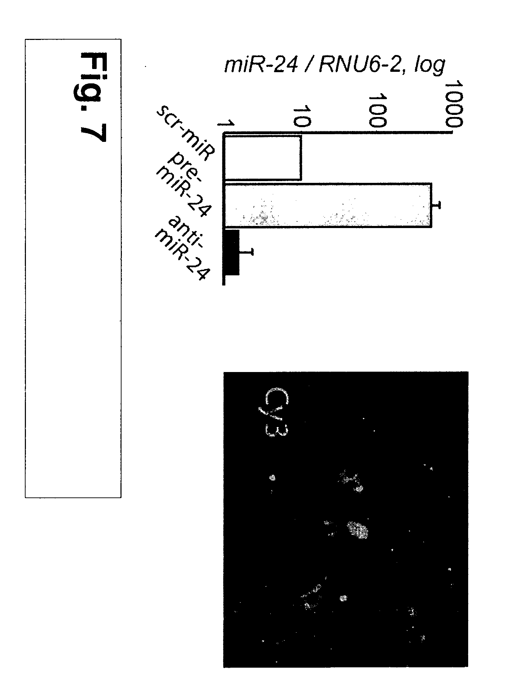

**Fig. 7** **displays transfection efficacy of miR-24 to endothelial cells.** On the left: Expression of miR-24 and RNU6b 72 h after transfection of scrambled miRNAs (scr-miR, 100nM), miR-24 precursors (pre-miR-24, 100nM) or miR-24 antagonists (anti-miR-24, 100nM) to human umbilical vein endothelial cells (HUVEC). On the right: Transfection of Cy3-labeled control miRNA precursors (72 h, 100 nM) to HUVECs.

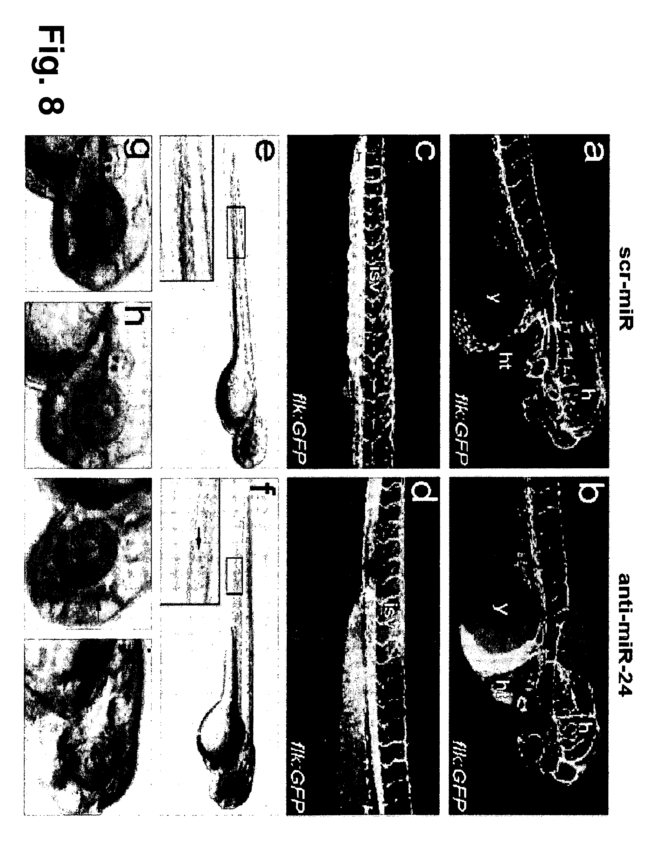

**Fig. 8** **displays modulation of miR-24 impacts on vascular homeostasis in zebrafish embryos.** Lateral views of (a,c,e,g,h) control (scrambled sequence)- and (b,d,f,i,j) miR-24-morpholino (MO)-injected tg(flk1:GFP) zebrafish embryos at 48 h post fertilization. Anti-miR morphants display normal trunk vasculature but vessels in the head display some degree of irregular patterning (arrow in (b)). Moreover hemorraghe is seen at random locations in different morphants (**f,i,,j**). H, head; ht, heart; y, yolk sac; isv, intersegmental vessels.

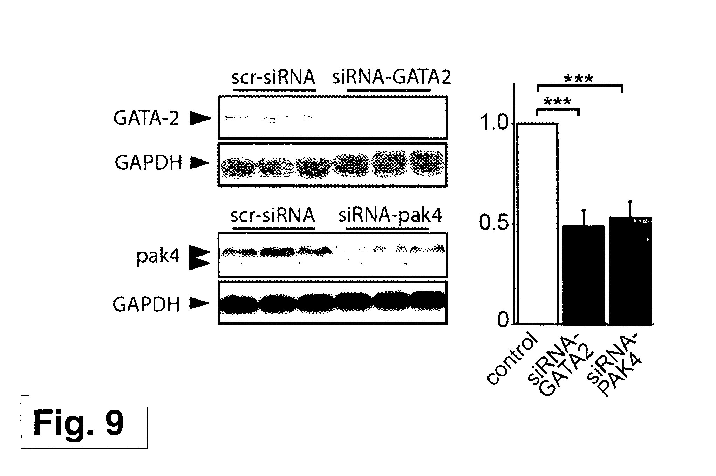

**Fig. 9** **displays siRNA-mediated repression of the miR-24 targets GATA2 and PAK4 in endothelial cells.** Western Blots of GATA2 (top) and PAK4 (bottom) 48 h after transfection of appropriate siRNA. Statistical summary (right). n=3 per group.

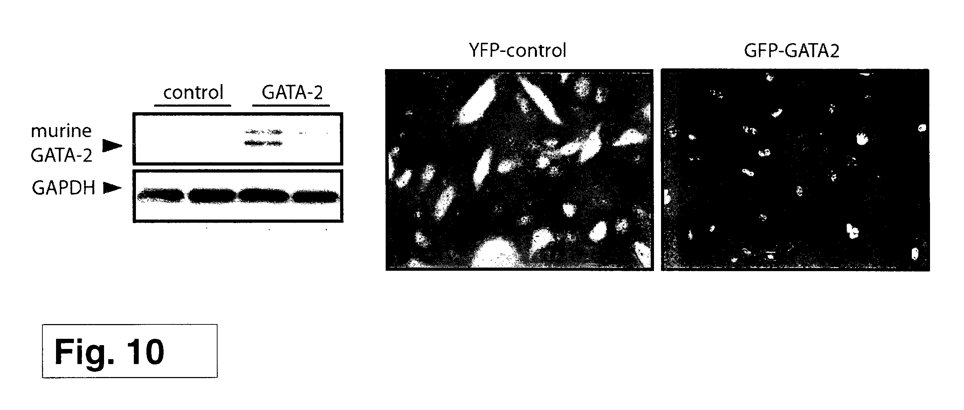

**Fig. 10** **displays adenoviral mediated overexpression of GATA2 in endothelial cells.** GATA2 expression after transfection of a murine GFP-GATA2 construct or an YFP-control construct to human umbilical vein endothelial cells.

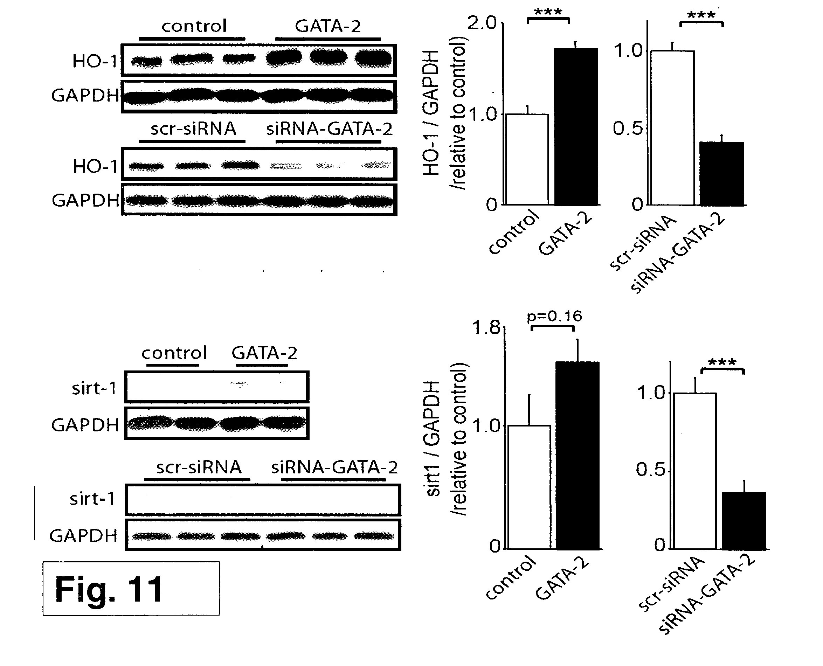

**Fig. 11** **displays heme oxygenase-1 and sirt1 regulation by GATA2 in endothelial cells.** Western blots of HO-1 (top) and sirt1 (bottom) after up- or down-regulation of GATA2 in HUVECs.

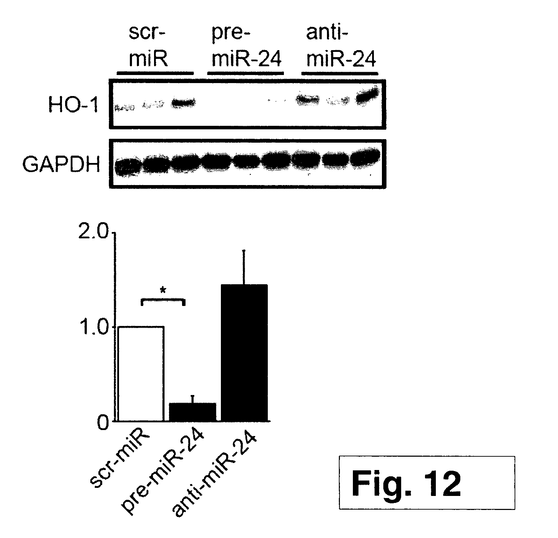

**Fig. 12** **displays miR-24 regulation of HO-1 expression in endothelial cells.**

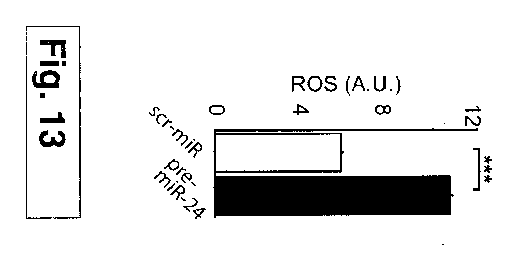

**Fig. 13** **displays increase of reactive oxygen species formation in endothelial cells by miR-24.** FACS-based analysis of reactive oxygen species (ROS) formation 72 h after transfection of synthetic miR-24 precursors (100 nM) to HUVECs.

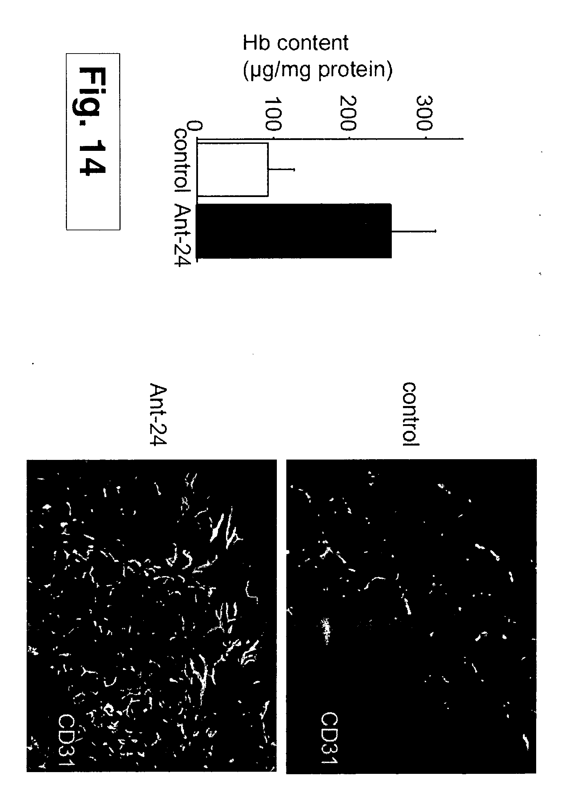

**Fig. 14** **displays increase of vascularization of implanted matrigel plugs by antagomir-24 treatment.** Hemoglobin (Hb) content and vascularization of matrigel plugs 14 days after implantation and treatment with two doses (day 0 and 2) of antagomir-24 or scrambled control. n=3-4 per group.

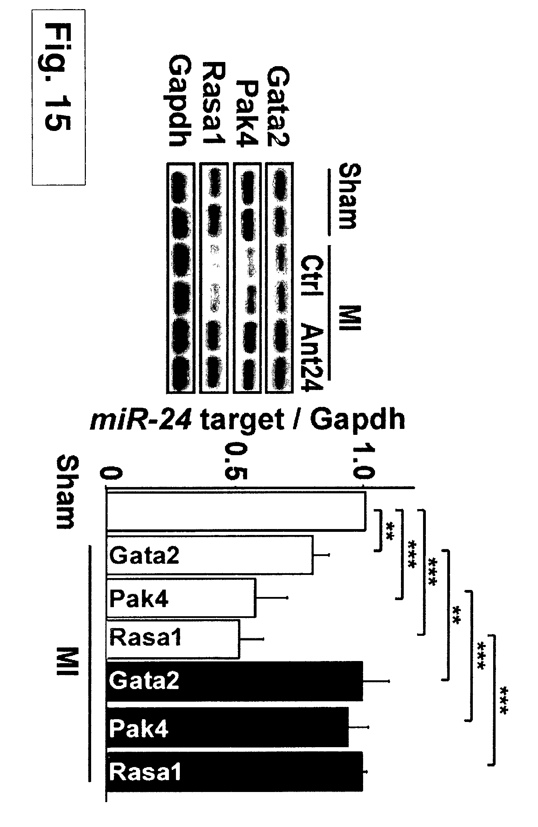

**Fig. 15** **displays an expression of the *miR-24* targets GATA2, PAK4, RASA1** in fractionated CD146+ cardiac endothelial cells obtained from Sham-operated mice and mice after MI post treatment with scrambled antagomir (Ctrl) or Antagomir-24 (Ant24). Statistical summary of protein expression data (right).

### Detailed description of the invention

A first aspect of the present invention relates to the use of GATA2, PAK4, RASA1 and/or a modulator, in particular an inhibitor of microRNA-24 (miR-24) for the manufacture of a medicament for the treatment and/or prevention of ischemia.

Further, the present invention relates to the use of GATA2, PAK4, RASA1 and/or a modulator, in particular an inhibitor of miR-24 for the manufacture of a medicament for the induction of angiogenesis.

The term "GATA2" as used herein is also known as GATA binding protein 2. GATA2 is a member of the GATA family of transcription factors that contain zinc fingers in their DNA binding domain. GATA2 is an endothelial-enriched transcription factor that regulates gene expression in hematopoietic cells and is expressed in non-hematopoietic embryonic stem cells and hematopoietic progenitors including early erythroid cells, mast cells, megakaryocytes, and endothelial cells.

The term "PAK4" as used herein is also known as P21(CDKN1A)-activated kinase 4. PAK4 is a member of the family of serine/threonine p21-activating kinases. PAK proteins are effectors that link Rho GTPases to cytoskeleton reorganization and nuclear signaling and serve as targets for the small GTP binding proteins Cdc42 and Rac. PAK4 interacts specifically with the GTP-bound form of Cdc42Hs and weakly activates the JNK family of MAP kinases. PAK4 is also a mediator of filopodia formation and may play a role in the reorganization of the actin cytoskeleton. As can be seen from these examples, PAK4 is implicated in a wide range of biological pathways.

The term "RASA1" as used herein is also known as RAS p21 protein activator 1 or RasGAP (Ras GTPase activating protein). RASA1 is a cytosolic human protein that is part of the GAP1 family of GTPase-activating proteins. RASA1 stimulates the GTPase activity of normal, but not oncogenic RAS p21. RASA1 transfers Ras from its active GTP-bound form to its inactive GDP-bound form by enhancing the endogenous GTPase activity of Ras by a C-terminal GAP domain. RASA1 is also active in mitogenic signal transmission towards downstream interacting partners by N-terminal SH2-SH3-SH2 domains. Mutations leading to changes in the binding sites of either protein are associated with basal cell carcinomas.

The term "microRNA-24" or "miRNA-24" as used herein refers to a family of small non-coding miRNA encoded by at least two distinct genes, MIR-24-1 and -2. The full-length miR-24-1 precursor is processed into two mature miRNAs, miR189 and miR-24. Van Rooij et al. (2006) identified miR-24 among a group of miRNAs upregulated in two independent mouse models of cardiac hypertrophy. Northern blot analysis showed increased expression of miR-24 in idiopathic end-stage failing human hearts. Overexpression of miR-24 in cultured rat cardiomyocytes resulted in hypertrophic growth, and cardiac overexpression of miR-24 in transgenic mice was embryonic lethal. In contrast to the invention, van Rooij did not report abnormal vessel architecture by manipulation of miR-24 expression. Wang et al. (2008) found that miR-24-1 regulated erythroid differentiation in erythroleukemic K562 cells and in CD34-positive human cord blood hematopoietic progenitor cells (HPCs) by downregulating expression of activin receptor ACVR1 B.

The term "modulator, in particular inhibitor of microRNA-24", also called miR-24 antagonist, as used herein refers to a substance or an effect that reduces or blocks activity of miRNA-24 on the DNA level, e.g. by modifying genetic translation; RNA level, e.g. by complementary oligonucleotides; and/or during RNA maturation, e.g. by splice modification. The inhibitor can act directly on miR-24 or indirectly via further compounds or effects within a signal pathway. Inhibitors of miR-24 can be naturally occurring, genetically modified or synthetic inhibitors, like morpholinos. The term "modulator", as used herein also comprises transfection of targets of miR-24.

The term "miRNA modulator" as used herein further comprises miRNA-mimetics, miRNA antagonists, e.g. antagomirs, or miRNA sponges, which may be used as effective tools and potentially therapeutically relevant approaches to treat diseases according to the invention. Delivering miRNA-mimics in disease states where specific miRNAs are repressed may improve or attenuate disease. In other diseases miRNAs get strongly upregulated and the goal in that case may be to lower their expression in selected cells by complementary RNA sequences. The synthetic reverse complement oligonucleotide approach can theoretically act at different levels to affect miRNA levels: (a) by binding to the mature miRNA within the RISC and acting as a competitive inhibitor; (b) by binding to the pre-miRNA and preventing its processing or entry into the RISC; (3) by interfering with the processing or export of the pre- or pri-miRNA from the nucleus.

Antagomirs are a novel class of chemically engineered cholesterol- conjugated single-strand RNA analogues that are efficient and specific silencers of endogenous microRNAs in vitro and in vivo. Inhibition of miRNAs can also be achieved with with antisense 2'-O-methyl (2'-OMe) oligoribonucleotides or by use of lentivirally or adenovirally expressed antagomirs. Furthermore, MOE (2'-O-methoxyethyl phosphorothioate) or LNA(locked nucleic acid (LNA) phosphorothioate chemistry)-modification of single-stranded RNA analogous can be used to inhibit miRNA activity.

In addition, endogenous miRNAs can be silenced by the use of miRNA sponges. In that case a single species of RNA is constructed, that contains multiple, tandem-binding sites for a miRNA seed family of interest. As various members of a miRNA seed family are targeted, the potential advantage of this approach is to more effectively influence disease pathways commonly regulated by this family of miRNAs. In principle it is possible to interfere with miRNA function by scavenging away the miRNA and thereby preventing it from binding its mRNA targets.

The binding of a miRNA with a specific mRNA target can also be prevented using an oligonucleotide with perfect complementary to the miRNA target sequence in the 3'-UTR of the mRNA, which thereby masks the binding site and prevents association with the miRNA. A further approach to inhibit miRNA function can be achieved by "erasers," in which expression of a tandem repeat of a sequence perfectly complementary to the target miRNA inhibits the endogenous miRNA function. Finally substances, molecules, drugs can be used to inhibit miRNA expression and biogenesis.

By delivering single-stranded oligonucleotides equivalent of the mature miRNA, an increase in the effective concentration of a reduced miRNA can be achieved through the use of synthetic RNA duplexes in which 1 strand is identical to the native miRNA. In this case, short double-stranded oligonucleotides are designed in which 1 strand is the mature miRNA sequence (guide strand) and a complimentary or partially complementary stand is complexed with the mature miRNA sequence (passenger strand). Finally substances, molecules, drugs can be used to increase miRNA expression and biogenesis

The term "ischemia" as used herein refers to a restriction or insufficiency in blood supply and/or flow to at least a part of the body. This restriction results in a lack or shortage of proper oxygen, like hypoxia, and nutrients, leading to tissue damage or dysfunction. Ischemia is caused by constriction or blockage of the supplying blood vessels. Ischemia of heart muscle produces angina pectoris. Ischemia may be caused by blood clots, congenital heart defects, head injury, stroke, hypoglycemia, tachycardia, atherosclerosis, hypotension, embolism, like thromboembolism, blood vessel constriction and outside compression of a blood vessel, e.g. by a tumor or in the case of the superior mesenteric artery syndrome, sickle cell, g-forces, which force the blood fluid to the body's extremities, like in acrobatics and military flying, localized extreme cold, such as by frostbite or improper cold compression therapy as well as other circumstances and conditions. The term "ischemia" comprises all types of ischemia independent of the pathomechanism, e.g. cardiac ischemia, bowel ischemia, ischemic colitis, mesenteric ischemia, cutaneous ischemia, and cerebral ischemia.

The term "angiogenesis" as used herein refers to a physiological process involving the growth of new blood vessels from pre-existing vessels. The term "angiogenesis" comprises vasculogenesis as spontaneous blood-vessel formation, intussusception as formation of new blood vessel by splitting off existing ones, and sprouting angiogenesis. The term "angiogenesis" herein refers also to its modern terminology, i.e. (1) vasculogenesis as formation of vascular structures from circulating or tissue-resident endothelial stem cells, which proliferate into de novo endothelial cells; (2) angiogenesis as formation of thin-walled endothelium-lined structures with/without muscular smooth muscle wall and pericytes; and (3) arteriogenesis as formation of medium-sized blood vessels are also included. Angiogenesis is a normal process in growth, development, and wound healing. It is also a fundamental step in the transition of tumors from a dormant state to a malignant state. The term "angiogenesis" as used herein summarizes all different types and modifications of arterial vessel growth, e.g. increase of capillary density and small vessel formation.

The inventors found that miR-24 is upregulated in endothelial cells, but not in cardiomyocytes post myocardial infarction and after hypoxic conditions (Fig. 1c-e). In the context of the invention, it was also found that miR-24 overexpression induces apoptosis specifically in endothelial cells, whereas miR-24 antagonism reduces apoptosis in this cell type. In contrast, miR-24 modulation is without effect on apoptosis in other cardiac cell types, such as neonatal cardiomyocytes, the cardiomyocyte cell line H9C2 or cardiac fibroblasts. Hypoxia-induced apoptosis of endothelial cells is attenuated by transfection of miR-24 antagonists, whereas additional transfection of miR-24 precursors exaggerates endothelial apoptosis (Fig. 2b).

The inventors show by different protein repression assays that miR-24 induces apoptosis specifically in endothelial cells by directly targeting the endothelial-enriched transcription factor GATA2 and the kinases PAK4 and RASA1 (Fig. 3). MiR-24-mediated reduction of PAK4 prevents BAD phosphorylation further contributing to endothelial apoptosis. GATA2 repression leads to a profound increase in apoptosis and impaired endothelial capillary network formation. GATA2 also regulates sirt1 and HO-1 for which the abbreviation HMOX-1 is also used in the present context.

In summary, miR-24 antagonism attenuates hypoxia-mediated endothelial apoptosis *in vitro.* GATA2, PAK4 and RASA1 and/or an inhibitor of miR-24 control a complex network of apoptotic and angiogenic programs and regulate capillary formation in endothelial cells. These important cellular characteristics impact on (neo-) vascularization *in vivo,* especially after ischemic events (Fig. 3h). Thus, miR-24 and its downstream targets in endothelial cells may serve as valuable therapeutic entry points to interfere with endothelial genetic programs leading to improved vascularization and cardiac performance after myocardial infarction. As shown by the data herein, GATA2, PAK4, RASA1 and/or an inhibitor of miR-24 can be used according to the invention for the manufacture of a medicament for the treatment and/or prevention of ischemia and/or the induction of angiogenesis.

In a preferred embodiment the medicament further comprises phosphorylated BAD, heme oxygenase 1 (HMOX-1), sirt1, bambi, esm1, and/or ntn4.

The term "BAD" as used herein refers to a distant member of the Bcl-2 family that promotes cell death. In contrast, phosphorylated BAD prevents apoptosis. The term "phosphorylated BAD" as used herein refers to at least one phosphorylation of BAD at any possible phosphorylation site, e.g. Ser112 and/or Ser136.

The term "heme oxygenase 1" or "HO-1", also called "HMOX-1", as used herein refers to an enzyme of the heme catabolism that cleaves heme to form biliverdin. Heme oxygenase activity is induced by its substrate heme and various non-heme substances. Heme oxygenase occurs as two isozymes, an inducible heme oxygenase-1 and a constitutive heme oxygenase-2 that belong both to the heme oxygenase family.

The term "sirt1" as used herein refers to a member of a family of currently seven proteins termed sirtuins. Sirt1 plays a central role in regulating cellular differentiation and senescence and controls metabolic pathways in response to nutrient availability in a wide variety of tissues.

The term "bambi" as used herein refers to a putative transmembrane glycoprotein related to the type I receptors of the transforming growth factor-beta (TGF-beta) family, whose members play important roles in signal transduction in many developmental and pathological processes. However, the encoded protein is a pseudoreceptor, lacking an intracellular serine/threonine kinase domain required for signaling.

The term "esm1" as used herein refers to a secreted protein, which is mainly expressed in the endothelial cells in human lung and kidney tissues. The expression of this gene is regulated by cytokines, and the transcript contains multiple polyadenylation signals and mRNA instability signals. Two transcript variants encoding different isoforms have been found for the coding gene of esm1.

HO-1, bambi, esm1, ntn4, sirt1 and phosphorylated BAD (phospho-BAD) are factors that indirectly regulate and modulate miR-24 effects.

All above mentioned downstream targets of miR-24, in particular GATA2, PAK4, RASA1 etc. may in particular exert their angiogenic properties after transfection of the respective cells or cell lines, e.g. via viral vectors or other commonly known transfection systems.

Genomic sequences of sirt1 and HO-1 display GATA2 binding sites within their promoter region, and enrichment of the respective DNA sequences after GATA2 immunoprecipitation was detected (Fig. 3g). Overexpression of GATA2 strongly induces protein expression of sirt1 and HO-1, whereas siRNA-mediated GATA2 silencing reduces sirt1 and HO-1 expression (Fig. 11). HO-1, which displayed no miR-24 binding site, is regulated by miR-24 via modulation of GATA2 (Fig. 12). HO-1 and sirt1 exert angiogenic, vasoprotective and anti-apoptotic actions in endothelium, and consequently, miR-24-mediated repression of HO-1 and sirt1 via GATA2 resulted in enhanced reactive oxygen species formation in endothelial cells (Fig. 13).

In a search for reversely regulated genes by GATA2 expression modulation, the inventors identified further genes, which are all enriched after GATA-2 CHIP (Fig. 3g). These identified genes code bambi, esm1 and ntn4 that are important proteins in angiogenesis. Like HO-1 and sirt1, the proteins bambi, esm1 and ntn4 show vasoprotective and anti-apoptotic actions in endothelium.

Endothelial miR-24 regulates a network of apoptotic and angiogenic proteins. Thus, miR-24 and its target proteins serve as specific therapeutic targets in the setting of ischemic diseases.

In another preferred embodiment GATA2, PAK4, RASA1, BAD, heme oxygenase 1 (HO-1 or HMOX-1), sirt1, bambi, esm1, and/or ntn4 is/are in the form of polypeptides, proteins, nucleic acids, in particular viral vectors comprising expression constructs, DNA and/or RNA, in particular siRNA and/or shRNA. Overexpression of microRNAs can in particular be achieved by local or systemic application of miRNA-precursor molecules / miRNA mimics or by viral overexpression of miRNAs by use of viral vectors.

In a preferred embodiment the inhibitor is an antagomir and/or an antisense oligonucleotide.

The term "antagomir" as used herein refers to a chemically engineered small RNA that is used to silence miR-24. The antagomir is complementary to the specific miRNA target with either mis-pairing or some sort of base modification. Antagomirs may also include some sort of modification to make them more resistant to degradation. In a preferred embodiment the antagomir is a chemically engineered cholesterol- conjugated single-stranded RNA analogue.

The term "antisense oligonucleotide" as used herein refers to a single strand of DNA or RNA that is complementary to a miR-24 and inactivates miR-24 by binding to it.

*In vivo,* injections of low concentrations of antagomir (about 5 mg/kg body weight) silence miR-24 expression specifically in the endothelial cell fraction, but not in cardiomyocyte that are only significantly repressed after application of high doses of antagomir (about 80 mg/kg body weight) (Fig. 4a,c). These low doses improve vascularization and heart function after myocardial infarction locally restricted in endothelial cells, i.e. undesired side effects are minimized. Therefore, in a preferred embodiment antagomir is applied in a low dose, preferably about 5 mg/kg body weight.

In a mouse model of myocardial infarction, blocking of endothelial miR-24 by systemic administration of a specific antagomir and/or antisense oligonucleotide enhances angiogenesis in the infarct zone and border zone. In addition, blocking of endothelial miR-24 preserves cardiac function measured e.g. by wet lung weight, systolic and diastolic left ventricular dilatation (Fig. 5f). Angiogenesis in the infarct zone and border zone comprises in a preferred embodiment an increase of hemoglobin content, invasion of cells as well as small vessel and capillary formation. No significant effects were observed in the remote region of the infarct.

Thus, miR-24 and its antagonists, antagomirs and/or antisense oligonucleotides, serve as spatially restricted therapeutic targets in the setting of ischemic diseases.

In a preferred embodiment the antagomir and/or the antisense oligonucleotide is essentially complementary to SEQ ID NO: 1 (miR-24).

In a further preferred embodiment the ischemia is associated with acute and/or chronic myocardial infarction, chronic heart failure, peripheral vascular occlusive disease, liver and/or kidney ischemia, stroke, Bowel ischemia and/or chronic ulcers of the skin and/or the mucosa.

A further aspect of the present invention relates to the use of a precursor of miR-24 (pre-miR-24) for the manufacture of a medicament for the treatment of angiogenesis associated with cancer.

Small solid tumors are not vascularized. To spread, they need to be supplied by blood vessels that bring oxygen and nutrients and remove metabolic wastes. Beyond the critical volume of about two cubic millimeters, oxygen and nutrients have difficulties diffusing to the cells in the center of the tumor, causing a state of cellular hypoxia that marks the onset of tumor angiogenesis. Therefore, blood vessel development is an important process in tumor progression and favors the transition from hyperplasia to neoplasia, i.e. the passage from a state of cellular multiplication to a state of uncontrolled proliferation characteristic of tumor cells. Neovascularization also influences the dissemination of cancer cells throughout the entire body eventually leading to metastasis formation.

The inventors found miR-24 to be a molecular factor involved in angiogenesis suitable as a treatment of angiogenesis associated with cancer.

A further aspect of the present invention relates to an *in vitro* method for diagnosing ischemia or prevalence for ischemia, comprising the steps of:
a) providing a test sample of a subject comprising endothelial cells;
b) identifying the amount of miR-24, GATA2, PAK4 and/or RASA1 in the test sample;
c) comparing the amount of miR-24, GATA2, PAK4 and/or RASA1 in the test sample with a control sample;
   wherein an up-regulation of miR-24 and/or a down-regulation of GATA2, PAK4 and/or RASA1 in the test sample indicates ischemia or prevalence for ischemia.

In the *in vitro* method according to the invention providing a test sample of a subject does not comprise the step of taking a sample from a human being, but the steps after taking the sample from a human being and the step of taking samples from non-human beings, preferably non-human mammalians. Providing a test sample or a cell culture includes all necessary or recommended preparation steps, like staining, centrifuging, isolating, purifying, filtrating, fixating, and precipitating.

MiR-24 overexpression inhibits angiogenesis selectively in endothelial cells and controls the apoptotic and angiogenic target proteins GATA2, PAK4 and RASA1. Thus, miR-24, GATA2, PAK4 and/or RASA1 serve as diagnostic markers in the setting of ischemic diseases (Fig. 8).

In a preferred embodiment the method according to the invention comprises further the steps of identifying and comparing the amount of phosphorylated BAD (phospho-BAD), heme oxygenase 1 (HO-1), sirt1, bambi, esm1, and/or ntn4, wherein a down-regulation of phospho-BAD, heme oxygenase 1 (HO-1), sirt1, bambi, esm1, and/or ntn4 in the test sample indicates ischemia or prevalence for ischemia (Fig. 3).

As indirect miR-24 targets bambi, esm1, ntn4, sirt1 and phospho-BAD serve as diagnostic markers in the setting of ischemic diseases (Fig. 11, 12 and 13). These additional targets make the diagnostic method according to the invention more reliable.

A further aspect of the present invention relates to a method for identifying a modulator of miR-24, GATA2, PAK4 and/or RASA1 comprising the steps of:
a) providing a cell culture expressing miR-24, GATA2, PAK4 and/or RASA1;
b) contacting a candidate substance with the cell culture;
c) assessing the activity and/or expression of miR-24, GATA2, PAK4 and/or RASA1;
d) comparing the expression and/or activity of miR-24, GATA2, PAK4 and/or RASA1 of step c) with the expression and/or activity in the absence of the candidate compound,
   wherein a difference in the expression and/or activity of miR-24, GATA2, PAK4 and/or RASA1 qualifies the candidate substance as a modulator of miR-24, GATA2, PAK4 and/or RASA1.

The "modulator" as used herein regulates and modifies expression and/or activity of miR-24, GATA2, PAK4, RASA1 and/or other miR-24 targets.

In a preferred embodiment the method according to the invention further comprises the steps of providing a cell culture expressing BAD, phospho-BAD, heme oxygenase 1 (HO-1), sirt1, bambi, esm1, and/or ntn4, assessing the activity and/or expression of BAD, phospho-BAD, heme oxygenase 1 (HO-1), sirt1, bambi, esm1, and/or ntn4, and comparing the expression and/or activity of BAD, phospho-BAD, heme oxygenase 1 (HO-1), sirt1, bambi, esm1, and/or ntn4. The additional miR-24 targets bambi, esm1, ntn4, sirt1, BAD and phospho-BAD make the screening method according to the invention more reliable.

A further aspect of the present invention relates to a kit comprising an antagomir and/or an antisense oligonucleotide essentially complementary to SEQ ID NO: 1.

In a preferred embodiment the kit according to the invention further comprises GATA2, PAK4, RASA1, BAD, phospho-BAD, HO-1, sirt1, bambi, esm1, and/or ntn4.

A further aspect of the present invention relates to an endothelial cell devoid of expressing functional miR-24. Functional miR-24 has its full activity without any reduction or modification.

A further aspect of the present invention relates to a non-human, transgenic animal comprising cells devoid of expressing functional miR-24.

A further aspect of the present invention describes a method for treating ischemia in a subject in need thereof, comprising the steps of:
a) identifying a subject suffering from ischemia;
b) inhibiting the expression of miR-24 and/or activating the expression of GATA2, PAK4 and/or RASA1 in endothelial cells of the subject.

A further aspect of the present invention describes a method for preventing ischemia in a subject which is at risk of developing ischemia, comprising the steps of:
a) identifying a subject which is at risk of developing ischemia;
b) inhibiting the expression of miR-24 and/or activating the expression of GATA2, PAK4 and/or RASA1 in endothelial cells of the subject.

### Results

### 1.1 Selective miR-24 upregulation in endothelial cells after myocardial infarction

Microarrays were used to identify deregulated miRNAs after myocardial infarction. MiR-24 was induced in total heart tissue after myocardial infarction *in vivo* (Fig. 1a). Induction was confirmed by miR-24-specific quantitative RT-PCR analysis (Fig. 1b). Using *in situ* hybridization only weak miR-24 signals were detected in normal myocardium, whereas the miR-24 hybridization signal was strongly increased in infarct and border zone areas of myocardium of mice fourteen days after myocardial infarction. Higher magnification revealed strong hybridization signals mainly in non-cardiomyocyte cells (Fig. 1 c). Specifically, when heart tissue was fractioned into cardiomyocytes and CD146 positive endothelial cells by magnetic affinity cell sorting, exclusive miR-24 upregulation was detected in endothelial cells after myocardial infarction, whereas miR-24 levels were unchanged in cardiomyocytes post-myocardial infarction (Fig. 1d). *In vitro,* hypoxic conditions (1 % O₂, 24 h) led to an increase of miR-24 expression in human umbilical vein endothelial cells (HUVEC), but not cardiomyocytes (Fig. 1e).

### 1.2 Activation of apoptotic programs in endothelial cells

To identify miR-24 function synthetic miR-24 precursors were overexpressed in various cardiac cell types. Transfection efficacy of miRNA precursors and antagonists was monitored by miRNA-specific quantitative realtime PCR (Fig. 7). MiR-24 overexpression induced apoptosis selectively in endothelial cells, whereas miR-24 antagonism reduced apoptosis in this cell type. In contrast, miR-24 modulation was without effect on apoptosis in other cardiac cell types, such as neonatal cardiomyocytes, the cardiomyocyte cell line H9C2 or cardiac fibroblasts (Fig. 2a).

In line, injection of a morpholino against miR-24 into tg(flk:GFP) zebrafish embryos that express GFP in the vasculature revealed no obvious changes in gross zebrafish morphology, but resulted in abnormal vessel architecture showing manipulation of miR-24 to result mainly in a vascular phenotype (Fig. 8). In contrast, cardiomyocyte-restricted overexpression of miR-24 was reported to induce cellular hypertrophy *in vitro* and to be embryonically lethal in mice (van Rooij et al., 2006).

Hypoxia-induced apoptosis of endothelial cells was attenuated by transfection of miR-24 antagonists, whereas additional transfection of miR-24 precursors exaggerated endothelial apoptosis (Fig. 2b). To identify proteins involved in miR-24 endothelial pro-apoptotic action, endothelial protein extracts were hybridized after transfection of scrambled miRNAs or synthetic miR-24 precursors to a protein-microarray spotted with antibodies for proteins involved in apoptosis. A number of pro-apoptotic proteins were upregulated, e.g. hypoxia-inducible factor-1 alpha and Fas, whereas despite lack of miR-24 binding sites strong reduction of Bad and hemeoxygenase 1 (HO-1) protein was found (Fig. 2c, d). Specifically, miR-24 overexpression led to a reduction of phosphorylated BAD (pBAD), whereas miR-24 antagonism induced the pBAD/BAD ratio (Fig. 2d). Next to the pro-apoptotic action of miR-24, impaired tube formation capacity on matrigel after miR-24overexpression in endothelial cells was observed (Fig. 2e).

### 1.3 MiR-24 coordinates a complex program for endothelial apoptosis and vascularization capacity by direct targets

To identify direct miR-24 targets that trigger endothelial apoptosis and impair tube formation capacity, bioinformatic target prediction tools were used and a substantial number of endothelial-expressed proteins with putative 3'UTR binding sites for miR-24 including the transcription factor GATA2, the p21-activated kinase PAK4 and the RAS p21 protein activator RASA1 were found. Transfection of primary endothelial cells with miR-24 precursors resulted in protein repression of GATA2, PAK4 and RASA1 (Fig. 3a). When the respective 3'UTR regions were fused to a luciferase reporter gene and luciferase activity was determined in cells transfected with synthetic miR-24 precursors, miR-24 significantly repressed luciferase activity, whereas other unrelated miRNAs (miR-22 or miR-21 0) had little effect (Fig. 3b). GATA2, PAK4 and RASA1 as direct targets of miR-24 were identified, and immunohistochemical analysis revealed predominant endothelial expression of these miR-24 targets within the mouse heart (Fig. 3c).

Transfection of small interference RNA (siRNA) against GATA2 or PAK4 to endothelial cells efficiently repressed GATA2 and PAK4 protein levels (Fig. 9). PAK4 leads to an increase in BAD phosphorylation and thus inactivation of the pro-apoptotic protein BAD. Repression of PAK4 resulted in a reduction of BAD phosphorylation (Fig. 3d) and increased the rate of apoptosis in endothelial cells (Fig. 3e). Similarly, GATA2 repression led to a profound increase in apoptosis and impaired endothelial tube formation (Fig. 3e and 3f).

To identify regulated targets of GATA2, global transcriptome analysis was combined after viral overexpression or silencing of GATA2 in endothelial cells with chromatin-immunoprecipitation (ChIP) procedures to enrich genomic sequences bound by GATA2. For GATA2 overexpression a murine GFP-GATA2 construct was used (Fig. 10). For reversely regulated genes after GATA2 modulation was searched and further genes important in angiogenesis were identified, e.g. bambi, esm1 and ntn4, which were all enriched after GATA-2 ChIP (Fig. 3g). Additionally, genomic sequences of sirt1 and heme oxygenase-1 (HO-1) displayed GATA2 binding sites within their promoter region, and enrichment of the respective DNA sequences after GATA2 immunoprecipitation was detected (Fig. 3g). In line, overexpression of a viral construct of GATA2 strongly induced protein expression of sirt1 and HO-1, whereas siRNA-mediated GATA2 silencing reduced sirt1 and HO-1 expression (Fig. 11). HO-1, which displayed no miR-24 binding site, was regulated by miR-24 via modulation of GATA2 (Fig. 12). HO-1 exerted vasoprotective and anti-apoptotic actions in endothelium, and consequently, miR-24-mediated repression of HO-1 via GATA2 resulted in enhanced reactive oxygen species formation in endothelial cells (Fig. 13).

In summary, a network of direct and indirect miR-24 targets was identified in endothelial cells regulating endothelial apoptosis and capillary formation, important cellular characteristics that impact on (neo-)vascularization *in vivo,* especially after ischemic events (Fig. 3h).

### 1.4 Specific targeting of cardiac endothelial cells by low antagomir concentrations

To study the effects of miR-24 on vascularization *in vivo,* chemically engineered cholesterol-conjugated single-strand RNA analogues (antagomirs) were injected to target miR-24 to mice (Fig. 4a). Cy3-labeled antagomirs were effectively taken up by endothelial cells *in vitro* (Fig. 4b). As *in vivo* mainly the endothelial cell fraction was targeted, and titration experiments with Cy-3 labeled antagomirs were performed. Injection of a Cy3-labeled antagomir at a low dose (5 mg/kg) mainly resulted in cellular uptake in cardiac vessels and the surrounding tissue, whereas injection of a high dose (80 mg/kg) led to a homogeneous stain of all cardiac cells including cardiomyocytes (Fig. 4b). Consequently, injections of low doses of an antagomir against miR-24 were found (5 mg/kg, day 0 and 2) to repress miR-24 mainly in fractionated CD146-positive endothelial cells obtained from total heart tissue, whereas miR-24 was only significantly repressed in cardiomyocyte after application of high doses (80 mg/kg, day 0 and 2) fourteen days after injection (Fig. 4a, c).

### 1.5 Antagomir-24 treatment improves vascularization and preserves cardiac function after myocardial infarction

Subsequently, effects of endothelial miR-24 antagonism in a mouse model of myocardial infarction were tested. Myocardial infarction led to an impairment of cardiac function and increase of systolic and diastolic left ventricular diameter fourteen days after intervention (Fig. 5a-d). In contrast, immediate treatment after myocardial infarction with an antagomir against miR-24 resulted in improved cardiac function and attenuation of left ventricular dilatation (Fig. 5a-d). Lung wet weight as a further marker for cardiac function was increased after myocardial infarction but attenuated by antagomir-24 treatment (Fig. 5e).

Treatment of mice after myocardial infarction with Antagomir-24 prevented downregulation of the miR-24 targets GATA2, PAK4, and RASA1 (Fig. 15). Immunohistochemical evaluation of CD31-positive cells revealed a significant induction of capillary density and small vessel formation in the border and infarct zone of the myocardial infarct after miR-24 antagonism. No significant effects were observed in the remote region of the infarct (Fig. 5f).

Enhanced invasion of cells and capillary formation as well as increased hemoglobin contents in implanted matrigel plugs in mice were also detected fourteen days after treatment with an antagomir against miR-24 suggesting general improvement of angiogenesis (Fig. 14). To exclude significant off-target effects and to confirm specificity of antagomir-24, antagomir against a scrambled sequence, which did not effect infarct healing or plug vascularization, was injected (Fig. 5a-f and Fig. 14).

### Methods

### 2.1 Cultivation of cardiovascular cells

Human umbilical vein endothelial cells (HUVECs) were cultured in EGM2 media supplemented with 20 % (v/v) fetal calf serum (FCS) and supplements (all reagents from Cambrex Lonza, UK). Cells were grown in a humidified atmosphere at 5 % CO₂ and 37°C. Neonatal rat cardiomyocytes and cardiac fibroblasts for apoptosis studies were isolated and cultured as described previously (Thum, 2008; Thum et al., 2007).

### 2.2 Fractionation of cardiac cell types from heart tissue

The thorax of mice was opened and the aorta was cannulated. After washing with 37°C PBS, the heart together with the cannula was removed and perfused with a collagenase solution for 5 min (Joklik MEM medium supplemented with 10 mM butanedione monoxime, 20 µM calcium chloride, 1 mg/ml collagenase II). The heart was placed in 37°C pre-warmed collagenase solution for further 25 min and was subsequently minced and filtered through a nylon mesh (200 µm pore size). Cardiomyocytes were separated by a sedimentation step as described (Thum, 2008). The non-cardiomyocyte cell fraction of the supernatant was incubated with anti-CD146 antibodies coupled to microbeads and subjected to magnetic affinity cell sorting according to the manufacturers' recommendations (Mouse CD146 microbead endothelial isolation kit, Miltenyi Biotec, Germany).

### 2.3 MiRNA/RNA isolation, miRNA-RT-PCR and global transcriptome analysis

RNA isolation was done with TRlzol reagent (Invitrogen, Karlsruhe, Germany) or the mirVana miRNA Isolation Kit (Ambion, USA) according to manufacturers' instructions. For detection of miRNAs in samples different TaqMan MicroRNA assays (Applied Biosystems, Foster City, USA) were applied (see Table M1). The small RNA molecule U6 small nuclear (Rnu6-2) was amplified as a control. RT-PCR analysis was performed in an ICycler (Bio-Rad, München, Germany). The organ panel for miR-24 expression analysis was purchased from BioCat (Heidelberg, Germany). To assess RNA integrity for downstream array analysis total RNA was subjected to capillary chromatography in an Agilent bioanalyzer 2100 (Santa Clara, USA). Gene array analysis was performed using the Affymetrix Genechip system and according to the manufacturer's instructions and using the following arrays: GeneChip 430 Human Gene 1.0ST arrays, Affymetrix Systems, USA. Further microarray analyses and data handling were performed using the XRAY software package (Biotiquesystems, USA).

### 2.4 In situ hybridization

*In situ* hybridization directed against miR-24 was carried out using a digoxigen (DIG)-labeled DNA oligonucleotide probe with approximately one-third of the residues substituted with locked nucleic acid (LNA) bases. Briefly, heart tissues were collected, immersed in 4 % formaldehyde solution for 24 h, then placed in 0.5 M sucrose for 48 h. Tissues were frozen in a dry-ice/ethanol bath, mounted for sectioning using a cryostat (Leica) and 10 µm sections were mounted on SuperFrost Plus glass slides (Thermo Fisher Scientific). Samples were fixed with formaldehyde-EDC. After fixation, the samples were exposed to 4 pmol of a LNA probe complementary to miR-24. The probe was hybridized overnight at a temperature of 20°C below the experimentally determined melting temperature, subsequently exposed to stringency washes and detected the miRNA using a series of enzymatic reactions with the product being a colorimetric precipitate of 5-bromo-4-chloro-3-indolyl phosphate (BCIP) and nitro blue tetrazolium (NBT). Slides were prepared for microscopy analysis and images captured on an Olympus BX50 microscope equipped with a DP70 camera and Olympus DP controller software.

### 2.5 Transfection assays

Transient liposomal transfection of small-inhibitory RNAs (siRNAs) or microRNAs was done according to manufacturers' instructions. Briefly, cells were splitted one day before transfection to reach 60-70 % confluence on day of transfection. Specific siRNAs/miRNAs and control siRNA/miRNA and Lipofectamine 2000 (Invitrogen, Germany) were mixed separately and incubated for 5 min with Opti-MEM I (Invitrogen) media. Complexes were added together and incubated for 20 min. Media was changed to antibiotic-free media before adding liposomal siRNA complexes (final concentration 150 nM for siRNA and 100nM for miRNAs). Cells were incubated for 4 h before changing the media to fresh medium. Silencing of proteins or miRNA targets was monitored 48 h (siRNA) or 72 h (miRNAs) post transfection by western blot analysis. Specific details about the used siRNAs and miRNAs are given in Table M2.

### 2.6 Detection Assays

**Annexin kit.** Apoptosis was measured with the Annexin-V-Fluos kit from Roche Diagnostics (Penzberg, Germany) according to manufacturers' instructions. FACS analysis was performed on a FACSCalibur (BD Biosciences, Franklin Lakes, USA).

**Western blotting.** Western blot analysis was performed with 20-40 µg total protein. Protein was blotted onto PVDF membrane in Mini Trans-Blot electrophoretic transfer cell (Bio-Rad, München, Germany). Afterwards different antigens were detected by appropriate antibodies (see Table M3).

**Apoptosis protein array.** Apoptosis array data were generated by applying a human apoptosis array kit (ARY009, R&D, USA). 200 µg protein from a pool of three samples were incubated with antibody-coated membranes following manufacturers' instructions. Various regulated proteins were then validated by Western blotting.

**ELISA.** For phospho-Bad (Ser112) detection in cell culture samples a PathScan Phospho-Bad (Ser112) Sandwich ELISA Kit (Cell Signaling, #7182) according to manufacturers' instructions was applied. Phospho-Bad levels were related to total BAD expression levels as obtained by Western Blotting.

**Detection of reactive oxygen species (ROS).** The redox-sensitive, cell-permeable fluorophore dihydroethidium (DHE) becomes oxidized in the presence of O₂⁻ to yield fluorescent ethidium. Thus, dye oxidation is an indirect measure of the presence of reactive oxygen intermediates. MiRNA-transfected HUVECs were incubated with DHE (2.5 µM) for 30 minutes. After washing, HUVECs were immediately analyzed with FACS (FACS Calibur, BD Bioscience).

**Tube formation assay.** Transfection of cultured cells was done as mentioned before. Then, cells were harvested and 15.000 cells were seeded on top of Matrigel coated chamber slides (Becton Dickinson, Germany). After 6 h and 24 h pictures were taken on a Zeiss Axiovison microscope (Jena, Germany).

### 2.7 MicroRNA targeting

**MicroRNA target prediction.** The microRNA databases and target prediction tools miRBase (http://microrna.sanger.ac.uk/), PicTar (http://pictar.mdc-berlin.de/) and TargetScan (http://www.targetscan.org/index.html) were used to identify potential microRNA targets. Specifically, targets with known expression in cardiovascular tissue were screened for.

**Luciferase reporter assays.** A luciferase reporter assay system was applied to validate potential miRNA targets. A putative 3'-UTR miRNA binding sequence was cloned into Spel and *Hind*III cloning site of pMIR-REPORT vector (Ambion). The resulting construct was co-transfected with miRNAs of interest and beta-galactosidase control plasmid (Promega) into HEK293 reporter cells in 48-wells by use of Lipofectamine 2000 (Invitrogen). 0.2 µg plasmid DNA and 100 nM miRNA were applied. Cells were incubated for 24 h before measuring luciferase and β-galactosidase activity (Promega). Experiments were done in quadruplicate.

**Immunofluorescence.** Frozen heart sections were acetone-fixed, washed and blocked with 5 % (v/v) donkey sera or MOM Mouse IgGs (for RASA1 stain) before addition of appropriate Alexa-conjugated secondary antibodies (Invitrogen). Slides were mounted in VECTASHIELD/DAPI (Linaris). Details about used antibodies are shown in Table M4.

**Viral transduction.** The original GFP-murine-GATA2 plasmid was from Novartis, Basel, Switzerland. N-terminal GFP-tagged GATA2 was subcloned in an appropriate adenoviral entry vector. For viral transduction experiments cells were grown to subconfluence and infected with viral particles. Multiplicity of infection (m.o.i.) was 40. An YFP control virus was also applied with same m.o.i.

**Chromatin immunoprecipitation.** Chromatin immunoprecipitation (ChIP) was used to detect protein-DNA interactions. First, protein G sepharose beads were blocked o/n at 4°C with 100 µg herring sperm DNA and 100 µg BSA per 100 µl 50 % (v/v) beads. HUVECs from confluent T75 flasks were first cross-linked by adding 1 % (v/v) formaldehyde for 10 min at 20°C. Cross-linking was stopped by addition of 2.5 M glycine to a final concentration of 125 mM for 5 min at 20°C. Cells were washed once with ice-cold PBS before scraped and harvested. The pellet was lysed in lysis buffer (85 mM KCI, 0.5 % (v/v) NP40 (Igepal CA-630, Sigma), 5 mM 1,4-piperazinediethanesulfonic acid (PIPES)), pH 8.0 on ice for 10 min with interval vortexing. Next, the lysate was sonified to yield DNA fragments from 100-1000 bp in length. Therefore, 12 intervals for each 30 seconds were applied (Bandelin Sonifier GM70, sonotrode MS72, cycle 0.5, maximum amplitude strength). Samples were kept on ice for the whole procedure. Afterwards, samples were centrifuged at maximum speed (10 min, 20°C). From these cleared lysates 100 µl aliquots were separately taken to measure sonification efficiency. Therefore samples were heated o/n at 65°C. Then, 100 µl dH₂O and proteinase K (0.5 mg/ml final) were added and samples were incubated for 3 h at 42°C. Finally, total DNA was purified with Qiagen PCR purification kit before agarose gel analysis.

To reduce non-specific background, cleared lysates were pre-cleared on 50 µl 50 % (v/v) blocked Protein G Sepharose beads (GE Healthcare) twice for 2 h at 20°C. Afterwards, samples were divided into 500 µl aliquots and filled up with 1 ml IP dilution buffer (0.01 % (w/v) SDS, 1.1 % (v/v) TritonX-100, 1.2 mM EDTA, 167 mM NaCl, 16.7 mM Tris-HCl, pH 8.1, 1 x protease blocker Roche). Samples were subjected to either immunoprecipitation with 5 µg GATA-2 antibody (Santa Cruz Biotechnology, sc-267X) or control mouse IgGs (Santa Cruz Biotechnology, sc-2025) o/n at 4°C. To block non-specific background 250 µg BSA and 24 µg herring sperm DNA were added. One sample with cell lysis and IP dilution buffer was used as mock control. The next day GATA-2/DNA cross-links were collected by 15 min incubation with 50 µl 50 % (v/v) Protein G beads at 4°C on a rotating wheel. Samples were centrifuged (3000 g, 1 min, 4°C) and beads were washed twice with dialysis buffer (2 mM EDTA, 0.2 % (v/v) sarcosyl, 50 mM Tris-HCl, pH 8.0, 1x protease blocker Roche) and four times with IP wash buffer (500 mM LiCl, 1 % (v/v) NP40, 1 % (v/v) Deoxycholate (DOC), 1x protease blocker Roche). Finally beads were washed twice with TE-buffer (1 mM EDTA, 10 mM Tris-HCl, pH 7.5). Antibody-GATA-2/DNA complexes were eluted from the beads by adding 150 µl elution buffer (100 mM NaHCO₃, 1 % (w/v) SDS, 1x protease blocker Roche). Samples were heated at 65°C and kept on a vortexing platform for 10 min. The elution step was repeated and combined eluates were reverse crosslinked by addition of 5 M NaCl (0.3 M final concentration) for 4 hours at 65°C. Samples were cooled on ice and subjected to RNA degradation by adding 10 µg RNase A and incubation for 30 min at 37°C. Next samples were mixed with 2.5 volumes 100 % (v/v) ethanol and incubated o/n at -20°C. On the following day DNA was pelleted (maximum speed, 20 min, 4°C) and protein was digested from the samples by addition of 100 µl TE buffer, 25 µl 5x proteinase K buffer (1.25 % (w/v) SDS, 25 mM EDTA, 50 mM Tris-HCl, pH 7.5) and 6.25 µl (20 µg/µl) proteinase K (2 h, 55°C). DNA was further extracted by phenol-chloroform-isoamylalcohol (PCI) extraction (1 volume sample on 1 volume PCI, maximum speed, 10 min, RT). The upper phase was mixed with 1 volume chloroform-isoamylalcohol and centrifuged (maximum speed, 10 min, 20°C). Again, the upper phase was taken and 1/10 volume 5 M NaCl and 5 µg glycogen were added. Samples were mixed with 2.5 V 100 % (v/v) ethanol and incubated o/n at -20°C. The next day DNA was pelleted (maximum speed, 10 min, 4°C) and washed with 70 % (v/v) ethanol. Pellets were air-dried and solved in 60 µl TE buffer before purification with Qiagen PCR purification kit.

For ChIP primer-design first 2000-2500 bp upstream promoter regions of candidate target genes by Ensembl Genome Browser were identified (http://www.ensembl.org/index.html). Subsequently, the promoter region for potential GATA2 binding sites by the use of ALLGEN-Promo was screened, and appropriate primer pairs that amplify potential GATA2 binding sites were selected. Subsequently, PCR analysis of chipped DNA fragments was done by mixing 2.5 µl sample, 2.5 µl 4 µM appropriate primer pairs, 10 µl HotStarTaq Mix (Qiagen) and applying the following protocol: 94°C 10 min, [94°C 1 min, 57°C 30 sec, 72°C 1 min] x 33, 72°C 10 min, 4°C hold. Used oligonucleotide primer sequences are given in Table M5.

### 2.8 In vivo studies

The studies conform to the Guide for the Care and Use of Laboratory Animals published by the US National Institutes of Health (NIH Publication No. 85-23, revised 1996).

**Zebrafish assays.** Care and breeding of zebrafish *danio rerio* were maintained at 28.5 °C and staged as described (Walker et al., 2007). Wildtype lines TU and TL and the following transgenic line were used: Tg(flk1:GFP). To inhibit pigmentation, 0.003 % 1-phenyl-2-thiourea was added to the embryo medium. Morpholino-modified oligonucleotides (MO) were obtained from Gene-Tools (Philomath, OR). Morpholino-modified oligonucleotides were directed against (dre-mir-24 5'-ctgttcctgctgaactgagccagca-3'). Embryos were injected at the 1-2 cell stage with 2-4 ng (500 µM) dre-mir-24. A standard control oligonucleotide (MO*-contronl*) (GENETOOLS, LLC) was injected at the same concentration as a negative control. Images of living embryos were acquired with Leica MZ FLIII. For confocal analyses, zebrafish embryos were fixed at 48 h in a 4 % paraformaldehyde solution overnight and embedded in 1.5 % LMW agarose. Confocal images of the head and tail were obtained with Leica TCS SP2 confocal laser scanning microscope. Series of sections with the optimized step size for the individual objectives were taken. The maximum projection algorithm of the Leica software was then used to calculate information sectored in different ranks to a two-dimensional projection.

**Antagomir injection.** Antagomirs were designed and provided by Regulus Therapeutics (USA) and as described (Krutzfeldt et al., 2005). Sequences were: Antagomir-24: 5'-CTGTTCCTGCTGAACTGAGCCA-chol-3', SEQ ID NO: 1 and scrambled Antagomir: 5'-ACAAACACCAUUGUCACACUCCA-chol-3', SEQ ID NO: 2. Antagomirs were diluted in nuclease-free water and 100 µl at concentrations of 5 mg/kg and 80 mg/kg were applied to mice via retroorbital injection.

**Myocardial infarction.** Mice (8-10 weeks) underwent coronary artery ligation for the production of myocardial infarction. Coronary ligation was carried out as described previously. Mice were anesthetized, placed on a heating pad, intubated and ventilated with a mixture of oxygen and isoflurane. After left lateral thoracotomy and exposure of the heart by retractors, the left anterior descending coronary artery (LAD) was permanently ligated. Successful production of myocardial infarction was checked by measurements of ST-elevation in ECGs as well as impaired left ventricular wall motion by echocardiography. Fourteen days after myocardial infarction, additional echocardiography and left ventricular catheterization measurements were performed and finally hearts were excised and cut into transverse sections. From the middle ring, 5 µm-sections were cut and stained with appropriate antibodies (see above). Left ventricular catheterization was performed via the right carotid artery using a miniaturized pressure sensing catheter. Cardiac dimensions and function were analyzed by pulse-wave Doppler echocardiography essentially.

**Matrigel implantation and determination of vascularization.** 300 µl Matrigel™ Basement Membrane Matrix High Concentration (Becton Dickinson, Germany) supplemented with 600 ng/ml bFGF, 300 ng/ml VEGF and 25 U/ml Heparin were injected subcutaneously into wildtype C57BL/6 mice and harvested two weeks later. Animals were treated post implantation with Antagomir-24 or a scrambled control antagomir (5mg/kg at day 0 and day 2) by retroorbital injection. Half of the plug were lysed in cell lysis buffer and samples were measured for hemoglobin amount with a Mouse Hemoglobin ELISA (#E-90HM, Immunology Consultants Laboratory Inc., USA). Hemoglobin amount was normalized to total protein. Additional plugs were frozen in TissueTec, sliced, stained with CD31 antibodies and investigated by fluorescence microscopy.

**Table M1: TaqMan miRNA detection assays**

| **miRNA** | **Reference** |
|---|---|
| hsa-miR-24 | #4373072, Applied Biosystems, Carlsbad, CA, USA |
| RNU6B | #4373381, Applied Biosystems, Carlsbad, CA, USA |

**Table M2: siRNAs and miRNAs**

| *siRNAs* | |
|---|---|
| **siRNA** | **Reference** |
| *GATA2* | sc-37228, Santa Cruz Biotechnology, Santa Cruz, CA, USA |
| *PAK4* | sc-39060, Santa Cruz Biotechnology, Santa Cruz, CA, USA |
| scrambled siRNA control-A | sc-37007, Santa Cruz Biotechnology, Santa Cruz, CA, USA |

| *miRNAs* | |
|---|---|
| **miRNA** | **Reference** |
| hsa-miR-24 | PM10737, Applied Biosystems, Carlsbad, CA, USA |
| hsa-anti-miR-24 | AM10737, Applied Biosystems, Carlsbad, CA, USA |
| hsa-miR-22 | PM11752, Applied Biosystems, Carlsbad, CA, USA |
| hsa-miR-210 | PM10516, Applied Biosystems, Carlsbad, CA, USA |
| pre-miR miRNA precursor molecules-negative control #2 | PM17111, Applied Biosystems, Carlsbad, CA, USA |

**Table M3: Antibodies applied in this work for Western blotting**

| **Antibody** | **Reference** | **Immunization** |
|---|---|---|
| GAPDH | ab8245, Abcam, Cambridge, MA, USA | mouse |
| GATA2 | arp31855, Aviva Systems Biology, San Diego, CA, USA | rabbit |
| HO-1 | AF3776, R&D Systems, Minneapolis, MN, USA | goat |
| PAK4 | ab19007, Abcam, Cambridge, MA, USA | rabbit |
| BAD | ab28840, Abcam, Cambridge, MA, USA | rabbit |
| RASA1/GAP | ab2922, Abcam, Cambridge, MA, USA | mouse |
| sirt1 | ab32441, Abcam, Cambridge, MA, USA | rabbit |

**Table M4: Antibodies applied in this work for immunofluorescence microscopy**

| *Primary antibodies* | | |
|---|---|---|
| **Antibody** | **Reference** | **Immunization** |
| CD31 | #2388 Serotec MCA | mouse |
| GATA2 | arp31855, Aviva Systems Biology, San Diego, CA, USA | rabbit |
| PAK4 | ab19007, Abcam, Cambridge, MA, USA | rabbit |
| RASA1/GAP | ab2922, Abcam, Cambridge, MA, USA | mouse |

| *Alexa-conjugated secondary antibodies* | | |
|---|---|---|
| **Antibody** | **Reference** | **Immunization** |
| anti-rat Alexa Fluor 488 | A21208, Invitrogen | donkey |
| anti-rabbit Alexa Fluor 594 | A21207, Invitrogen | donkey |
| anti-mouse Alexa Fluor 594 | A21203, Invitrogen | donkey |

**Table M5: Primers used for ChIP-PCR**

| **Promoter region** | **primer (forward, reverse)** | **Product size [bp]** |
|---|---|---|
| BMP and activin membrane-bound inhibitor (BAMBI) | forward: 5'-tctcaggttttggagggaga-3' (SEQ ID NO:5) | 259 |
| | reverse: 5'-ggccgagactgacactcaat-3' (SEQ ID NO:6) | |
| Endothelial cell specific molecule 1 (ESM1) | forward: 5'- caagtgatatgccagggtca -3' (SEQ ID NO:7) | 136 |
| | reverse: 5'- tggttgttttgcatgaggac -3' (SEQ ID NO:8) | |
| Heme oxygenase 1 (HO-1) | forward: 5'- catcaccagacccagacaga-3' (SEQ ID NO:9) | 133 |
| | reverse: 5'- aaggccgactttaagggaag-3' (SEQ ID NO:10) | |
| Netrin 4 (NTN4) | forward: 5'-gagccagttattcagcaaagaaa-3' (SEQ ID NO:11) | 180 |
| | reverse: 5'-atgcagaggccatgctaatc-3' (SEQ ID NO:12) | |
| Sirtuin 1 (sirt1) | forward: 5'-ggagtcacagtgtgccagaa-3' (SEQ ID NO:13) | 201 |
| | reverse: 5'-ccttcctttctagcgtgagc-3' (SEQ ID NO:14 | |

### Reference List

Bartel, D. P. MicroRNAs: Genomics, biogenesis, mechanism, and function. Cell 116, 281-297, 2004
Care, A., et al. MicroRNA-133 controls cardiac hypertrophy. Nat Med 13, 613-618, 2007
Hill, J.A., et al. Cardiac Plasticity. N Engl J Med 358, 1370-80, 2008
Krutzfeldt, J., et al. Silencing of microRNAs in vivo with 'antagomirs'. Nature 438, 685-689, 2005
Thum, T., et al. MicroRNAs in the human heart: a clue to fetal gene reprogramming in heart failure. Circulation 116, 258-267, 2007
Thum, T. MicroRNA-21 contributes to myocardial disease by stimulating MAP kinase signalling in fibroblasts. Nature 456, 2008
van Rooij, E., et al. A signature pattern of stress responsive microRNAs that can evoke cardiac hypertrophy and heart failure. PNAS 103, 18255-60, 2006
Walker, M.B., et al. phospholipase C, beta 3 is required for Endothelin1 regulation of pharyngeal arch patterning in zebrafish. Developmental Biology 304, 194-207, 2007
Wang, S., et al. The Endothelial-Specific MicroRNA miR-126 Governs Vascular Integrity and Angiogenesis. Developmental Cell 15, 261-71, 2008
Wang, Q., et al. MicroRNA miR-24 inhibits erythropoiesis by targeting activin type I receptor ALK4. Blood 111, 588-595, 2008.

## Claims

1. Use of a modulator in particular an inhibitor of microRNA-24 (miR-24), GATA2, PAK4 and/or RASA1 for the manufacture of a medicament for the treatment and/or prevention of ischemia.

2. Use of a modulator in particular an inhibitor of microRNA-24 (miR-24), GATA2, PAK4 and/or RASA1 for the manufacture of a medicament for the induction of angiogenesis.

3. Use of claim 1 or 2, wherein the medicament further comprises phosphorylated BAD, heme oxygenase 1 (HO-1 or HMOX-1), sirt1, bambi, esm1, and/or ntn4.

4. Use of any of the preceding claims, wherein GATA2, PAK4, RASA1, BAD, heme oxygenase 1 (HO-1 or HMOX-1), sirt1, bambi, esm1, and/or ntn4 is/are in the form of polypeptides, proteins, nucleic acids, in particular viral vectors comprising expression constructs, DNA and/or RNA, in particular siRNA and/or shRNA.

5. Use of any of the preceding claims, wherein the inhibitor is an antagomir and/or an antisense oligonucleotide.

6. Use of claim 5, wherein the antagomir and/or the antisense oligonucleotide is essentially complementary to SEQ ID NO: 1.

7. Use of any of the preceding claims, wherein the ischemia is associated with acute and/or chronic myocardial infarction, chronic heart failure, peripheral vascular occlusive disease, liver and/or kidney ischemia, stroke, Bowel ischemia and/or chronic ulcers of the skin and/or the mucosa.

8. Use of a precursor of miR-24 (pre-miR-24) for the manufacture of a medicament for the treatment of angiogenesis associated with cancer.

9. An *in vitro* method for diagnosing ischemia or prevalence for ischemia, comprising the steps of:
a) providing a test sample of a subject comprising endothelial cells;
b) identifying the amount of miR-24, GATA2, PAK4 and/or RASA1 in the test sample;
c) comparing the amount of miR-24, GATA2, PAK4 and/or RASA1 in the test sample with a control sample;
wherein an up-regulation of miR-24 and/or a down-regulation of GATA2, PAK4 and/or RASA1 in the test sample indicates ischemia or prevalence for ischemia.

10. The method of claim 9, further comprising the steps of identifying and comparing the amount of phosphorylated BAD, heme oxygenase 1 (HO-1 or HMOX-1), sirt1, bambi, esm1, and/or ntn4, wherein a down-regulation of phosphorylated BAD, heme oxygenase 1 (HO-1 or HMOX-1), sirt1, bambi, esm1, and/or ntn4 in the test sample indicates ischemia or prevalence for ischemia.

11. A method for identifying a modulator of miR-24, GATA2, PAK4 and/or RASA1 comprising the steps of:
a) providing a cell culture expressing miR-24, GATA2, PAK4 and/or RASA1;
b) contacting a candidate substance with the cell culture;
c) assessing the activity and/or expression of miR-24, GATA2, PAK4 and/or RASA1;
d) comparing the expression and/or activity of miR-24, GATA2, PAK4 and/or RASA1 of step c) with the expression and/or activity in the absence of the candidate compound,
wherein a difference in the expression and/or activity of miR-24, GATA2, PAK4 and/or RASA1 qualifies the candidate substance as a modulator of miR-24, GATA2, PAK4 and/or RASA1.

12. The method of claim 11, further comprising the steps of providing a cell culture expressing BAD, phosphorylated BAD, heme oxygenase 1 (HO-1 or HMOX-1), sirt1, bambi, esm1, and/or ntn4, assessing the activity and/or expression of BAD, phosphorylated BAD, heme oxygenase 1 (HO-1 or HMOX-1), sirt1, bambi, esm1, and/or ntn4, and comparing the expression and/or activity of BAD, phosphorylated BAD, heme oxygenase 1 (HO-1 or HMOX-1), sirt1, bambi, esm1, and/or ntn4.

13. A kit comprising an antagomir and/or an antisense oligonucleotide essentially complementary to SEQ ID NO: 1.

14. The kit of claim 13, further comprising GATA2, PAK4, RASA1, BAD, phosphorylated BAD, heme oxygenase 1 (HO-1 or HMOX-1), sirt1, bambi, esm1, and/or ntn4.

15. An endothelial cell devoid of expressing functional miR-24.

16. A non-human, transgenic animal comprising cells devoid of expressing functional miR-24.

17. A modulator in particular an inhibitor of microRNA-24 (miR-24), GATA2, PAK4 and/or RASA1 for use as a medicament for the treatment and/or prevention of ischemia.

18. A modulator in particular an inhibitor of microRNA-24 (miR-24), GATA2, PAK4 and/or RASA1 for use as a medicament for the induction of angiogenesis.
